(19) Europäisches Patentamt
European Patent Office
Office européen des brevets

(11) EP 4 332 095 A1

(12) **EUROPEAN PATENT APPLICATION**
published in accordance with Art. 153(4) EPC

(43) Date of publication:
**06.03.2024 Bulletin 2024/10**

(21) Application number: **22795682.8**

(22) Date of filing: **22.04.2022**

(51) International Patent Classification (IPC):
***C07D 307/46*** (2006.01) ***C07D 307/68*** (2006.01)
***C07D 407/04*** (2006.01) ***C07B 61/00*** (2006.01)

(52) Cooperative Patent Classification (CPC):
**C07D 307/46; C07D 307/68; C07D 407/04;**
C07B 61/00

(86) International application number:
**PCT/JP2022/018549**

(87) International publication number:
**WO 2022/230769 (03.11.2022 Gazette 2022/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.04.2021 JP 2021077077**
**30.04.2021 JP 2021077087**
**30.04.2021 JP 2021077963**
**01.06.2021 JP 2021092374**
**01.06.2021 JP 2021092375**
**10.06.2021 JP 2021097495**

(71) Applicant: **Mitsubishi Chemical Corporation**
**Chiyoda-ku**
**Tokyo 100-8251 (JP)**

(72) Inventors:
- **FURUYA, Mitsunori**
**Tokyo 100-8251 (JP)**
- **AOSHIMA, Takayuki**
**Tokyo 100-8251 (JP)**

(74) Representative: **Vossius & Partner**
**Patentanwälte Rechtsanwälte mbB**
**Siebertstrasse 3**
**81675 München (DE)**

# (54) METHOD FOR PRODUCING FURAN DERIVATIVE INCLUDING 5-HYDROXYMETHYLFURFURAL (5-HMF), FURAN DERIVATIVE, SOLUTION CONTAINING SAID FURAN DERIVATIVE, AND METHOD FOR PRODUCING FURAN DICARBOXYLIC ACID OR FURAN DICARBOXYLIC ACID ESTER

(57) The present invention addresses the problem of providing a method by which 5-hydroxymethylfurfural can be inexpensively produced from glucose with a high yield and a high selectivity. This problem is solved by a method of producing 5-hydroxymethylfurfural, which method includes the step of bringing glucose into contact with an isomerization catalyst and a dehydration catalyst in a biphasic solvent of water and a water-nonmiscible organic solvent.

EP 4 332 095 A1

**Description**

TECHNICAL FIELD

The present invention relates to a method of producing 5-hydroxymethylfurfural (hereinafter, also referred to as "5-HMF") by bringing a mixed catalyst, which is obtained by mixing an isomerization catalyst and a dehydration catalyst, into contact with glucose in a biphasic solvent of a water-nonmiscible organic solvent and water.

The present invention also relates to: a method of producing a formylfurancarboxylic acid acetal, which is an intermediate in the production of a dicarboxylic acid ester or a dicarboxylic acid that has a furan skeleton derived from a biomass raw material; a formylfurancarboxylic acid acetal; and a solution containing the same.

Further, the present invention relates to: a method of producing a furan derivative, which is an intermediate in the production of a furandicarboxylic acid or a furandicarboxylic acid ester that is derived from a biomass raw material; the furan derivative; and a solution containing the same.

Moreover, the present invention relates to a method of producing a furandicarboxylic acid or a furandicarboxylic acid ester that is derived from a biomass raw material.

BACKGROUND ART

In recent years, there is a need to address the issues of fossil fuel depletion and global-scale environmental burden of increasing carbon dioxide in the atmosphere. Because of such social demands, the development of a process for producing fuels and various useful chemicals from biomass raw materials grown under the current global atmospheric environment is attracting attention and being actively pursued.

Such a method of utilizing a biomass raw material is expected to be a means for replacing petroleum raw materials. For example, as the production of plant raw materials can be dispersed and diversified across various places, there is an advantage that a very stable supply of raw materials is achieved. In addition, since fuels can be produced and consumed in the atmosphere without consuming fossil fuels, the difference in mass balance between absorption and emission and carbon dioxide becomes relatively balanced. Therefore, the use of biomass raw materials is advantageous in that it potentially holds the feasibility of a recycling society, which cannot be expected with the use of fossil fuels, and offers an extremely high industrial utility value.

In a series of catalytic reactions that induce a starting material of a fuel or a chemical raw material from a biomass raw material, 5-hydroxymethylfurfural (5-HMF) is an important intermediate. Generally, 5-HMF is produced by an acid-catalyzed intramolecular dehydration reaction of fructose having a hexose skeleton that is used as a raw material. 5-HMF serves as a raw material of 2,5-dimethylfuran (DMF) and 2,5-furandicarboxylic acid (FDCA). In recent years, DMF has been attracting attention as a biofuel alternative to fossil fuels, and FDCA is, as an alternative to terephthalic acid constituting polyethylene terephthalate, converted into polyethylene furanoate (PEF) by condensation-polymerization with ethylene glycol.

Patent Document 1 discloses a method of producing 5-HMF from a hexose. Specifically, 5-HMF is obtained by allowing glucose and a poly(styrenesulfonic acid) $AlCl_3$ (PSSA-$AlCl_3$) water-soluble complex catalyst to come into contact and react with each other in a biphasic solvent of water and a polar organic solvent (methyl isobutyl ketone (MIBK)/2-BuOH) at 150°C. The conversion rate and the yield of this method are 95% and 45%, respectively.

Meanwhile, Non-patent Document 1 discloses a method of obtaining 5-HMF by dissolving fructose in a solvent having a *N*-methylpyrrolidone:water ratio of 70:30 and subsequently passing the resultant through a flow reactor filled with a cation exchange resin (DIAION RCP160M) catalyst. The conversion rate and the yield of this method are both 91%. Fructose is usually produced by isomerization of glucose. Non-patent Document 2 discloses a method of isomerizing glucose using a glucose isomerase. Since this isomerization reaction is an equilibrium reaction, the yield of this method is 57%. In this manner, fructose in circulation is produced by separation and recovery of fructose generated from glucose at a rate of a little over 50%.

Non-patent Document 3 discloses a method of obtaining 5-HMF by bringing glucose into contact with sodium chloride and a cation exchange resin (DIAION RCP160M) catalyst in a biphasic solvent of water, *N*-methylpyrrolidone, and methyl isobutyl ketone. This method is described to have a yield of 84% and a selectivity of 90%.

On another front, in a series of catalytic reactions that induce a useful chemical from a biomass raw material, a process performed through a compound having a furan ring has been proposed as a particularly important process from the standpoint of its functionality, versatility of use, environmental load, and economic efficiency. For example, methods based on the following reactions have been proposed as methods of producing a furandicarboxylic acid or an ester thereof, which is the production target of the present invention.

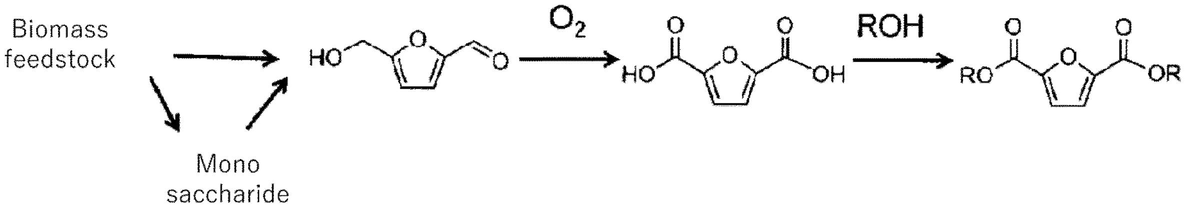


As concrete production methods, for example, Patent Document 2 proposes a method of producing hydroxymethylfurfural (HMF) from a monosaccharide, such as glucose or fructose, in the presence of hydrated niobic acid, and Patent Documents 3 and 4 propose methods of producing a furandicarboxylic acid (FDCA) or an ester thereof by way of an oxidation reaction of HMF. However, in these methods disclosed in Patent Documents 3 and 4 in which a furandicarboxylic acid or an ester thereof is produced from HMF by a direct oxidation reaction, since HMF has reactive functional groups such as an aldehyde group and a hydroxyl group and thus has a low thermal stability, side reactions such as polymerization and ring-opening reactions occur concurrently. Not only these side reactions lead to the formation of oligomers and polymers such as humin, levulinic acid, and the like and thereby reduce the reaction yield, but also the oligomers and the polymers adhere to the inside of a reactor and a pipe to cause a reduction in the heat transfer efficiency of the reactor and clogging of the pipe, making it difficult to achieve stable long-term operation. These problems are prominent especially in the production of a furandicarboxylic acid or an ester thereof under a high-HMF-concentration condition; therefore, the production is forced to be carried out under a low-HMF-concentration condition, which presents a major problem for an industrial process that requires economic efficiency.

On the other hand, as a high-productivity process that can efficiently yield a furandicarboxylic acid (FDCA) or an ester thereof even in a high-HMF-concentration condition, Patent Document 5 proposes a method of producing a furandicarboxylic acid (FDCA) or an ester thereof using a cyclic acetal of HMF as an intermediate. However, in this production method as well, there is still a problem that the yield of FDCA is reduced when the concentration of the cyclic acetal of HMF is increased. This is because the acetal group of the cyclic acetal is unlikely to undergo an oxidation reaction in the production method disclosed in Patent Document 5. In order to facilitate the oxidation reaction of the acetal group, it is necessary to increase the amount of a catalyst to be used and/or to perform the reaction under a high-pressure condition using a high-oxygen-concentration gas. The reaction under such a stringent oxidizing condition have problems in that it not only requires the use of a high-pressure equipment with a high-oxygen-concentration gas and thus inflates the construction cost of a production facility, but also leads to an increase in the production cost since a diol required for obtaining the cyclic acetal of HMF is partially consumed by oxidation. In addition, since a complex purification process is required for avoiding the contamination of a decomposition product of this diol into a product and recovered diol, there is a problem of a further increase in the production cost.

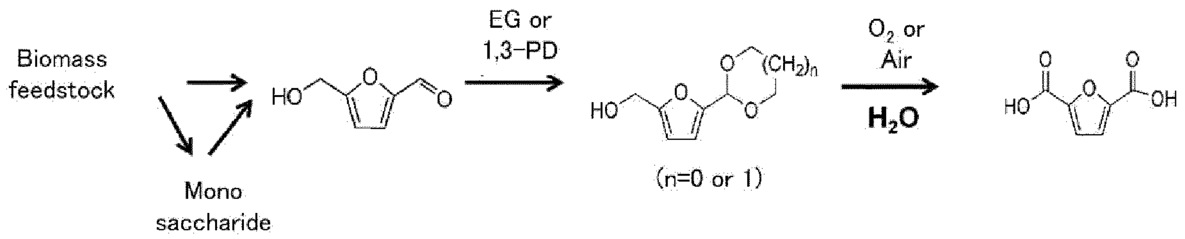


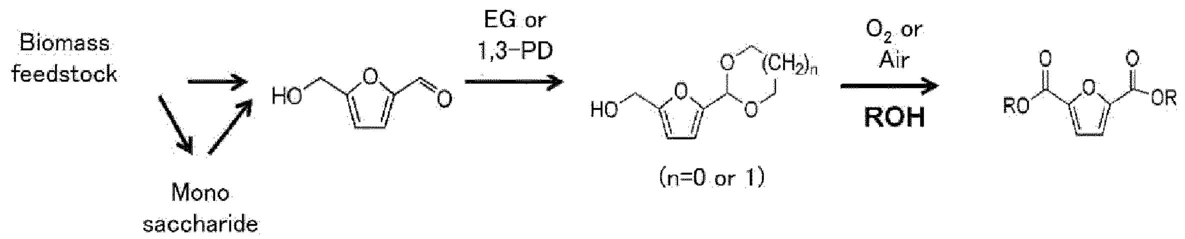


RELATED ART DOCUMENTS

PATENT DOCUMENTS

[Patent Document 1] WO 2019/089448
[Patent Document 2] Japanese Unexamined Patent Application Publication No. 2009-215172
[Patent Document 3] Japanese Patent No. 5217142
[Patent Document 4] WO 2015/155784
[Patent Document 5] Japanese Unexamined Patent Application Publication No. 2018-39778

NON-PATENT DOCUMENTS

[Non-patent Document 1] Chemical Engineering & Processing, Process Intensification, 138, 2019, 65-72
[Non-patent Document 2] Yoshiyuki Takasaki, "Development of Isomerized Sugar by Glucose Isomerase", The Society of Synthetic Organic Chemistry, Japan, 1980, Vol. 38, No. 6, p.538-545
[Non-patent Document 3] Royal Society of Chemistry Advances, 2020, 10, 9492-9498

SUMMARY OF THE INVENTION

PROBLEMS TO BE SOLVED BY THE INVENTION

Methods of producing 5-HMF from fructose with a good yield have been reported in Non-patent Document 1. In these production methods, however, the step of isomerizing glucose into fructose is indispensable. For example, in the case of obtaining fructose from glucose by the method of Non-patent Document 2 or the like and subsequently producing 5-HMF by the method of Non-patent Document 1, the cumulative yield stays at a little over 50% or so.

In the production of 5-HMF from glucose by the method disclosed in Patent Document 1, the yield is about 45%. When 5-HMF is produced from glucose by the method of Non-patent Document 3, the yield is improved. However, condensation products and oligomers such as humin as well as decomposition products such as formic acid and levulinic acid are formed by side reactions, and this results in a low 5-HMF selectivity of about 90%. In addition, these by-products and decomposition products may cause coloring. When such coloring occurs, a complex purification process such as an adsorption-decolorization process is required for the color removal, which leads to an increase in the production cost.

Moreover, when these by-products and decomposition products adhere to the inside of a reactor, they cause a reduction in the heat transfer efficiency of the reactor and pipe clogging, making it difficult to achieve stable long-term operation.

In the case of producing 5-HMF from glucose by the method disclosed in Non-patent Document 3, sodium chloride used in this method leads to deactivation (capping) of an acidic group of a cation exchange resin; therefore, there is also a problem of a decrease in the catalyst life. Moreover, since the corrosion of a reactor is accelerated, a reactor made of a material having a higher corrosion resistance, such as a glass-lined reactor or a Hastelloy reactor, is required. In other words, the method disclosed in Non-patent Document 3 has a problem of inflating the construction cost of a reaction facility.

As described above, the reaction yield and selectivity are still insufficient in the production of 5-HMF directly from glucose, and many problems remain to be solved in terms of productivity and long-term stable operation.

In this respect, an object of the present invention is to provide a method by which 5-HMF can be inexpensively produced from glucose with a high yield and a high selectivity. More particularly, an object of the present invention is to provide a process of producing 5-HMF from glucose, which has a basic configuration that, by bringing a mixed catalyst obtained by mixing an isomerization catalyst and a dehydration catalyst into contact with glucose in a biphasic solvent of a water-nonmiscible organic solvent and water, (1) while isomerizing glucose into fructose using the isomerization catalyst, the thus partially generated fructose is quickly and selectively converted into 5-HMF by the dehydration catalyst, and (2) 5-HMF generated during the reaction is distributed as needed in an organic solvent phase contained in a biphasic solvent of water and an organic solvent separated into two phases with water, in such a manner that the generated 5-HMF does not cause a decomposition reaction such as by-production of formic acid and levulinic acid, or a polymerization reaction such as formation of humin or oligomer that causes coloring (first problem).

Further, as described above, Patent Documents 3 to 5 propose methods of producing FDCA or an ester thereof by way of an oxidation reaction of HMF; however, from the industrial standpoint, there are still problems to be solved in terms of productivity and production cost.

Another object of the present invention is to solve the above-described problems in a method of producing FDCA or an ester thereof by way of an oxidation reaction of HMF, and provide a novel intermediate through which the method of producing FDCA or an ester thereof can be carried out with a high yield under more advantageous reaction conditions in terms of production cost (second problem).

MEANS FOR SOLVING THE PROBLEMS

The present inventors intensively studied the above-described first problem and consequently discovered that, by allowing a mixed catalyst of an isomerization catalyst and a dehydration catalyst to react with glucose and distributing the resulting 5-HMF in an organic solvent that is separated into two phases with water, side reactions such as the above-described decomposition reaction and polymerization reaction can be inhibited, and 5-HMF can be produced with a high yield and a high selectivity (94% to 95%).

That is, a first gist of the present invention encompasses the following.

<I>

[1] A method of producing 5-hydroxymethylfurfural, the method including the step of allowing glucose and a mixed catalyst obtained by mixing an isomerization catalyst and a dehydration catalyst to come into contact and react with each other in a biphasic solvent of a water-nonmiscible organic solvent and water.

[2] A method of producing 5-hydroxymethylfurfural, the method including the contact step of bringing glucose into contact with an isomerization catalyst and a dehydration catalyst in a biphasic solvent of water and a water-nonmiscible organic solvent,

wherein the biphasic solvent contains 80 parts by mass or less of an alkali metal salt or an alkaline earth metal salt with respect to 100 parts by mass of glucose.

[3] The method of producing 5-hydroxymethylfurfural according to [1] or [2], wherein the isomerization catalyst is a metal oxide.

[4] The method of producing 5-hydroxymethylfurfural according to any one of [1] to [3], wherein the dehydration catalyst is a solid acid.

[5] The method of producing 5-hydroxymethylfurfural according to any one of [1] to [4], wherein

the isomerization catalyst has a specific surface area of 100 $m^2/g$ or more, and
the dehydration catalyst has a specific surface area of 40 $m^2/g$ or more.

[6] The method of producing 5-hydroxymethylfurfural according to any one of [1] to [5], wherein a ratio between a value obtained by multiplying the specific surface area and the amount of the isomerization catalyst and a value obtained by multiplying the specific surface area and the amount of the dehydration catalyst is 1:20 to 20:1.

[7] The method of producing 5-hydroxymethylfurfural according to any one of [1] to [6], wherein a mixing ratio (mass ratio) of the isomerization catalyst and the dehydration catalyst is 1:3 to 3:1.

[8] The method of producing 5-hydroxymethylfurfural according to any one of [1] to [7], wherein

the isomerization catalyst is aluminum oxide, and
the dehydration catalyst is an ion exchange resin.

[9] The method of producing 5-hydroxymethylfurfural according to any one of [1] to [8], wherein the organic solvent is hydrophobic and at least one selected from the group consisting of ethers, ketones, aromatic hydrocarbons, and saturated aliphatic hydrocarbons.

[10] The method of producing 5-hydroxymethylfurfural according to any one of [1] to [9], wherein the organic solvent has a boiling point of 70°C or higher under atmospheric pressure.

[11] The method of producing 5-hydroxymethylfurfural according to any one of [1] to [10], wherein the organic solvent has an inorganicity/organicity ratio of 0.10 to 1.00.

[12] The method of producing 5-hydroxymethylfurfural according to any one of [1] to [11], wherein the organic solvent is at least one selected from the group consisting of methyltetrahydropyran (MTHP), methyl isobutyl ketone (MIBK), and methyl ethyl ketone (MEK).

[13] The method of producing 5-hydroxymethylfurfural according to any one of [1] to [12], wherein a mass ratio of the organic solvent and water is 1:1 to 4:1.

[14] The method of producing 5-hydroxymethylfurfural according to any one of [1] to [13], wherein the temperature in the reaction is 120°C or higher.

<II>

[1] A method of producing 5-hydroxymethylfurfural, the method including:

the step of allowing glucose and a mixed catalyst obtained by mixing an isomerization catalyst and a dehydration catalyst to come into contact and react with each other in a biphasic solvent of a water-nonmiscible organic solvent and water; and
the oil-water separation step of separating a reaction solution obtained by the reaction into an organic solvent phase containing 5-hydroxymethylfurfural generated by the reaction, and an aqueous phase containing a saccharide and the mixed catalyst that are contained in a solution obtained after the reaction.

[2] A method of producing 5-hydroxymethylfurfural, the method including:

the contact step of bringing glucose into contact with an isomerization catalyst and a dehydration catalyst in a biphasic solvent of water and a water-nonmiscible organic solvent; and
the oil-water separation step of separating a reaction solution obtained by the reaction into an organic solvent phase containing 5-hydroxymethylfurfural generated by the reaction, and an aqueous phase containing a saccharide and the mixed catalyst that are contained in a solution obtained after the reaction.
wherein the biphasic solvent contains 80 parts by mass or less of an alkali metal salt or an alkaline earth metal salt with respect to 100 parts by mass of glucose.

[3] The method of producing 5-hydroxymethylfurfural according to [1] or [2], wherein

the isomerization catalyst is a metal oxide, and
the dehydration catalyst is a solid acid.

[4] The method of producing 5-hydroxymethylfurfural according to any one of [1] to [3], wherein

the isomerization catalyst has a specific surface area of 100 $m^2/g$ or more, and
the dehydration catalyst has a specific surface area of 40 $m^2/g$ or more.

[5] The method of producing 5-hydroxymethylfurfural according to any one of [1] to [4], wherein a ratio between a value obtained by multiplying the specific surface area and the amount of the isomerization catalyst and a value obtained by multiplying the specific surface area and the amount of the dehydration catalyst is 1:20 to 20:1.
[6] The method of producing 5-hydroxymethylfurfural according to any one of [1] to [5], wherein a mixing ratio (mass ratio) of the isomerization catalyst and the dehydration catalyst is 1:3 to 3:1.
[7] The method of producing 5-hydroxymethylfurfural according to any one of [1] to [6], wherein

the isomerization catalyst is aluminum oxide, and
the dehydration catalyst is an ion exchange resin.

[8] The method of producing 5-hydroxymethylfurfural according to any one of [1] to [7], wherein the organic solvent is hydrophobic and at least one selected from the group consisting of ethers, ketones, aromatic hydrocarbons, and saturated aliphatic hydrocarbons.
[9] The method of producing 5-hydroxymethylfurfural according to any one of [1] to [8], wherein the organic solvent has a boiling point of 70°C or higher under atmospheric pressure.
[10] The method of producing 5-hydroxymethylfurfural according to any one of [1] to [9], wherein the organic solvent has an inorganicity/organicity ratio of 0.10 to 1.00.
[11] The method of producing 5-hydroxymethylfurfural according to any one of [1] to [10], wherein the organic solvent is at least one selected from the group consisting of methyltetrahydropyran (MTHP), methyl isobutyl ketone (MIBK), and methyl ethyl ketone (MEK).
[12] The method of producing 5-hydroxymethylfurfural according to any one of [1] to [11], including the step of dissolving (distributing) and recovering 5-hydroxymethylfurfural generated by the reaction in the organic solvent phase obtained by the oil-water separation step.
[13] The method of producing 5-hydroxymethylfurfural according to any one of [1] to [12], including the step of recovering the saccharide and the mixed catalyst that are contained in the solution obtained after the reaction, along with the aqueous phase obtained by the oil-water separation step.
[14] The method of producing 5-hydroxymethylfurfural according to any one of [1] to [13], wherein a mass ratio of the organic solvent and water is 1:1 to 4:1.
[15] The method of producing 5-hydroxymethylfurfural according to any one of [1] to [14], wherein the temperature in the reaction is 120°C or higher.

Further, the present inventors intensively studied the above-described second problem and consequently discovered a method of producing a novel intermediate through which a method of producing FDCA or an ester thereof can be carried out, thereby completing the present invention. In addition, the present inventors discovered that the above-described problem can also be solved by employing a method that includes specified production steps, thereby completing the present invention. By using the intermediate and the specific steps according to the present invention, not only FDCA or an ester thereof but also diformylfuran and a formylfurancarboxylic acid having an asymmetric substituent can be produced with a high yield and a high selectivity.
That is, a second gist of the present invention encompasses the following.
<III>

[1] A method of producing a furan derivative having an acetal group and a carbonyl group, the method including the oxidation step of bringing hydroxymethylfurfural acetal into contact with an oxidation catalyst.
[2] The method according to [1], wherein the furan derivative is a compound represented by Formula (1):

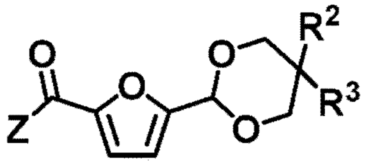
(1)

(wherein, Z represents a hydrogen atom or a hydroxyl group; and $R^2$ and $R^3$ are independently selected from a hydrogen atom, an alkyl group having 1 to 6 carbon atoms, and an aryl group having 6 to 12 carbon atoms that optionally has an alkyl substituent).
[3] The method according to [1] or [2], wherein, in a product obtained after the oxidation step, the concentration of the furan derivative in all compounds having a furan skeleton is 70% by mole or higher.
[4] The method according to any one of [1] to [3], wherein the oxidation catalyst is a metal catalyst.
[5] A furan derivative represented by Formula (1):

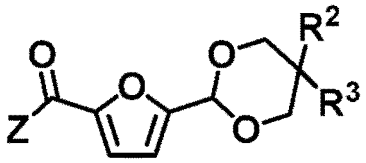
(1)

(wherein, Z represents a hydrogen atom or a hydroxyl group; and $R^2$ and $R^3$ are independently selected from a hydrogen atom, an alkyl group having 1 to 6 carbon atoms, and an aryl group having 6 to 12 carbon atoms that optionally has an alkyl substituent).
[6] A solution containing the furan derivative according to [5], wherein the concentration of the furan derivative in all compounds having a furan skeleton is 70% by mole or higher.
[7] The solution according to [6], wherein the furan derivative is a compound represented by Formula (2):

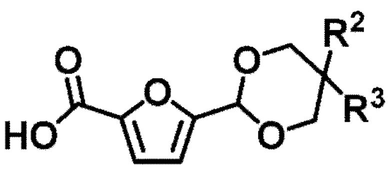
(2)

(wherein, $R^2$ and $R^3$ are independently selected from a hydrogen atom, an alkyl group having 1 to 6 carbon atoms, and an aryl group having 6 to 12 carbon atoms that optionally has an alkyl substituent).

<IV>

[1] A method of producing a furan derivative, the method including the deprotection step of deprotecting a formylfurancarboxylic acid acetal or an ester thereof to synthesize a furan derivative,

wherein the furan derivative is at least one selected from the group consisting of a formylfurancarboxylic acid and an ester thereof.

[2] The method according to [1], wherein the formylfurancarboxylic acid acetal or ester thereof is a compound represented by Formula (3):

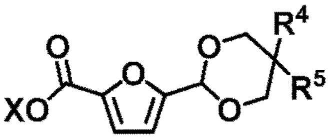 (3)

(wherein, X, $R^4$, and $R^5$ are independently selected from a hydrogen atom, an alkyl group having 1 to 6 carbon atoms, and an aryl group having 6 to 12 carbon atoms that optionally has an alkyl substituent).

[3] The method according to [1] or [2], wherein, in a product obtained after the deprotection step, the concentration of the formylfurancarboxylic acid and ester thereof in all compounds having a furan skeleton is 70% by mole or higher.

[4] The method according to any one of [1] to [3], including the recovery step of recovering a diol from a reaction solution after the deprotection step.

[5] The method according to [4], wherein the diol is a diol having 2 to 10 carbon atoms.

[6] A solution containing compounds having a furan skeleton, wherein the concentration of a formylfurancarboxylic acid and an ester thereof in all of the compounds having a furan skeleton is 70% by mole or higher.

<V>

[1] A method of producing a furandicarboxylic acid or an ester thereof, the method including the oxidation step of bringing a composition containing a furan derivative into contact with an oxidation catalyst, wherein

the furan derivative is at least one selected from the group consisting of diformylfuran, a formylfurancarboxylic acid, and an ester thereof, and

the concentration of the furan derivative in all compounds having a furan skeleton that are contained in the composition containing the furan derivative is 70% by mole or higher.

[2] The method according to [1], wherein the oxidation catalyst is a metal catalyst.

[3] The method according to [1] or [2], wherein the reaction temperature in the oxidation step is 50°C or lower.

<VI>

[1] A method of producing a furandicarboxylic acid or a furandicarboxylic acid ester from hydroxymethylfurfural, the method including the following production steps (i) to (iv):

(i) the acetalization step of acetalizing hydroxymethylfurfural to synthesize hydroxymethylfurfural acetal;

(ii) the first oxidation step of oxidizing the hydroxymethylfurfural acetal to synthesize a formylfurancarboxylic acid acetal or an ester thereof;

(iii) the deprotection step of deprotecting the formylfurancarboxylic acid acetal or ester thereof to synthesize a formylfurancarboxylic acid or an ester thereof; and

(iv) the second oxidation step of oxidizing the formylfurancarboxylic acid or ester thereof.

[2] The method of producing a furandicarboxylic acid or a furandicarboxylic acid ester according to [1], wherein the formylfurancarboxylic acid acetal or ester thereof is a compound represented by Formula (3):

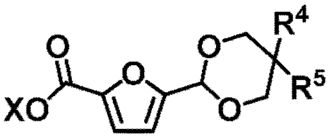 (3)

(wherein, X, $R^4$, and $R^5$ are independently selected from a hydrogen atom, an alkyl group having 1 to 6 carbon

atoms, and an aryl group having 6 to 12 carbon atoms that optionally has an alkyl substituent).

[3] The method of producing a furandicarboxylic acid or a furandicarboxylic acid ester according to [1] or [2], wherein, in the step (i), an organic solvent selected from toluene and methyltetrahydropyran is used.

[4] The method of producing a furandicarboxylic acid or a furandicarboxylic acid ester according to any one of [1] to [3], including the recovery step of recovering a diol from a reaction solution after the step (iii).

[5] The method of producing a furandicarboxylic acid or a furandicarboxylic acid ester according to [4], wherein the diol is a diol having 2 to 10 carbon atoms.

EFFECTS OF THE INVENTION

According to the first gist of the present invention, by bringing glucose into contact with a mixed catalyst obtained by mixing an isomerization catalyst and a dehydration catalyst, and isomerizing glucose into fructose, the thus partially generated fructose can be converted into 5-HMF by the dehydration catalyst more quickly and selectively. In addition, by promptly distributing the thus generated 5-HMF in an organic solvent, deterioration of the product can be inhibited, so that 5-HMF can be inexpensively produced from glucose with a higher yield and a higher selectivity than a conventional method. In other words, according to this method, 5-HMF can be produced directly from a glucose raw material with a high yield and a high selectivity without the need for a preliminary preparation process of producing a fructose intermediate from the glucose raw material, so that not only the construction cost of a production facility can be reduced, but also complicated operational management can be avoided.

Further, according to the second gist of the present invention, in a method of producing FDCA or an ester thereof by an oxidation reaction of HMF, a novel intermediate through which the method of producing FDCA or an ester thereof can be carried out can be provided. Moreover, by carrying out the method through this intermediate, not only the pressure and the temperature in the oxidation reaction can be reduced but also the use of a high-pressure equipment using a high-oxygen-concentration gas is no longer required, contributing to a reduction in the production cost. Furthermore, FDCA or an ester thereof can be produced with a high yield.

MODE FOR CARRYING OUT THE INVENTION

Representative modes of carrying out the present invention will now be described concretely; however, the present invention is not limited to the below-described modes within the gist of the present invention.

In the present specification, those ranges that are stated with "to" before and after numerical or physical property values each denote a range that includes the respective values stated before and after "to".

<First Gist>

One embodiment (first gist) of the present invention is a method of producing 5-hydroxymethylfurfural, which method includes the contact step of bringing glucose into contact with an isomerization catalyst and a dehydration catalyst in a biphasic solvent of water and a water-nonmiscible organic solvent.

<Reaction Method>

The reactions according to the present embodiment are performed by, for example, a batch method in which a substrate, a solvent, and catalysts are put into a reactor, or a flow method in which a solution is circulated through a reactor (e.g., a fluidized bed or an immobilized bed) into which catalysts are put. Alternatively, a CSTR continuous stirred tank-type reaction method is preferably employed as well. Examples of a liquid-contact material of the reactor, its flow channels, and the like include, but not particularly limited to: glass, stainless steel (SUS), iron, and other metals. A material made of glass or stainless steel (SUS) is preferably used because of its corrosion resistance and high energy transfer efficiency. Further, from the standpoint of the cost of production equipment, it is preferred to use a material made of stainless steel (SUS).

<Organic Solvent>

The organic solvent used in the present embodiment is not particularly limited as long as it is hydrophobic and nonmiscible with water, and it may be a mixture as well. In other words, the organic solvent may be any organic solvent that is separated into two phases when mixed with water.

The boiling point of the organic solvent used in the present embodiment under atmospheric pressure is not particularly limited. The boiling point is usually 70°C or higher, preferably 75°C or higher, more preferably 80°C or higher. On the other hand, an upper limit of the boiling point is usually 250°C or lower, preferably 200°C or lower, more preferably

150°C or lower. By using an organic solvent whose boiling point is in this range, the reaction conversion rate can be improved. In addition, the load in the distillation-purification of recovered organic solvent can be reduced.

The organic solvent used in the present embodiment is not particularly limited. Examples thereof include: ethers having 4 to 20 carbon atoms, such as tetrahydropyran, methyltetrahydropyran, dipropyl ether, diisopropyl ether, methyl-tert-butyl ether, butyl ether, pentyl ether, hexyl ether, octyl ether, nonyl ether, decyl ether, ethylene glycol dimethyl ether, ethylene glycol diethyl ether, diethylene glycol diethyl ether, and triethylene glycol dimethyl ether; ketones having 4 to 20 carbon atoms, such as 2-butanone (methyl ethyl ketone), 2-pentanone, 3-pentanone, 2-hexanone, 3-hexanone, cyclohexanone, 4-methyl-2-pentanone (methyl isobutyl ketone), 2-heptanone, 5-methyl-2-hexanone, 2,4-dimethyl pentanone, 5-nonanone, 4-decanone, 5-decanone, 2-undecanone, 4-undecanone, 3-dodecanone, 2-tridecanone, 2-tetradecanone, 4-tetradecanone, 2-pentadecanone, 3-pentadecanone, 7-pentadecanone, 2-hexadecanone, 3-hexadecanone, 4-hexadecanone, 6-hexadecanone, 2-heptadecanone, 4-heptadecanone, 9-heptadecanone, 3-octadecanone, and acetophenone; esters, such as ethyl acetate, propyl acetate, and butyl acetate; monoalcohols having 4 to 20 carbon atoms, such as 1-butanol, 2-butanol, 2-methyl-1-propanol, 2-methyl-2-propanol, 1-pentanol, 2-pentanol, 3-pentanol, 3-methyl-1-butanol, 2,2-dimethyl-1-propanol, 1-hexanol, 2-hexanol, 3-hexanol, 2-methyl-1-pentanol, 1-heptanol, 2-heptanol, 1-octanol, 2-octanol, 3-octanol, 2-ethyl-1-hexanol, 1-nonanol, 1-decanol, 1-undecyl alcohol, 1-lauryl alcohol, 1-tridecyl alcohol, 1-tetradecanol, 1-pentadecyl alcohol, 1-hexadecanol, cis-9-hexadecen-1-ol, 1-heptadecanol, 1-octadecanol, 16-methylheptadecen-1-ol, nonadecyl alcohol, and arachidyl alcohol; saturated aliphatic hydrocarbons having 3 to 12 carbon atoms, such as pentane, hexane, cyclohexane, heptane, octane, nonane, decane, dodecane, and isododecane; aromatic hydrocarbons, such as toluene, xylene, trimethylbenzene, 1,2,3,4-tetrahydronaphthalene, and 1-methylnaphthalene; lactones, such as γ-butyrolactone and γ-valerolactone; and halogenated hydrocarbons, such as dichloromethane, chloroform, dichloroethane, tetrachloroethane, and hexachloroethane.

Thereamong, the organic solvent is preferably at least one selected from the group consisting of ethers, ketones, esters, lactones, aromatic hydrocarbons, halogenated hydrocarbons, and saturated aliphatic hydrocarbons. The organic solvent is more preferably at least one selected from the group consisting of ethers, ketones, aromatic hydrocarbons, and saturated aliphatic hydrocarbons, particularly preferably an ether or a ketone.

The ether is more preferably a cyclic ether. Specific examples of the cyclic ether include methyltetrahydropyran (MTHP) and tetrahydropyran (THP). Therebetween, methyltetrahydropyran (MTHP) is more preferred since it has a high boiling point under atmospheric pressure and improves the reaction conversion rate in a short time, so that a high reaction selectivity can be obtained.

The ketone is more preferably methyl isobutyl ketone (MIBK), methyl ethyl ketone (MEK), or diethyl ketone. Methyl isobutyl ketone (MIBK) or methyl ethyl ketone (MEK) is still more preferred since it has a high boiling point under atmospheric pressure and improves the reaction conversion rate in a short time, so that a high reaction selectivity can be obtained.

As an aromatic compound, xylene, toluene, or benzene is more preferred. Toluene is still more preferred since it has a high boiling point under atmospheric pressure and improves the reaction conversion rate in a short time, so that a high reaction selectivity can be obtained.

These organic solvents may be used singly, or in any combination of two or more thereof at any ratio.

In the present embodiment, by using the above-described organic solvent having the above-described boiling point, the reaction conversion rate is improved in a short time, and 5-HMF can thus be obtained with a high reaction selectivity. In addition, since the generated 5-HMF can be efficiently extracted, the recovery rate of 5-HMF by subsequent oil-water separation can be improved.

The inorganicity and the organicity of solvents are proposed by Organic Conception Diagram ("Systematic Organic Qualitative Analysis", Atsushi Fujita, Kazamashobo Co., Ltd. (1974)). The organicity and the inorganicity are calculated based on the values predetermined for the functional groups constituting an organic compound, and the properties of the organic compound are characterized by the ratio of the organicity and the inorganicity.

The above-described organic solvent of the present embodiment has an inorganicity/organicity ratio of preferably 0.10 to 1.00 as defined in Organic Conception Diagram ("Systematic Organic Qualitative Analysis", Atsushi Fujita, Kazamashobo Co., Ltd. (1974)). A lower limit of the inorganicity/organicity ratio is more preferably 0.20 or higher, and an upper limit is more preferably 0.90 or lower. By selecting a solvent whose inorganicity/organicity ratio is in this range, the affinity is improved for not only hydrocarbon groups but also hydroxy groups and aldehyde groups, so that 5-HMF can be efficiently extracted. Table 1 below shows the inorganicity (I), the organicity (O), and the ratio thereof (I/O) for major organic solvents.

[Table 1]

| | Solvent name | Inorganicity(I) | Organicity(O) | I/O |
|---|---|---|---|---|
| Aromatic | Toluene | 15 | 140 | 0.11 |
| | Benzene | 15 | 120 | 0.13 |
| Ether | Methyltetrahydropyran | 30 | 110 | 0.27 |
| | Diethyl ether | 20 | 80 | 0.25 |
| | Tetrahydropyran | 30 | 80 | 0.38 |
| | Dioxane | 50 | 80 | 0.63 |
| Ketone | Acetone | 65 | 60 | 1.08 |
| | Methyl ethyl ketone | 65 | 80 | 0.81 |
| | Methyl isobutyl ketone | 65 | 110 | 0.59 |
| Alcohol | Ethanol | 100 | 40 | 2.50 |
| | Isopropyl alcohol | 100 | 50 | 2.00 |
| | Propanol | 100 | 60 | 1.67 |
| | Butanol | 100 | 80 | 1.25 |
| Ester | Ethyl acetate | 85 | 80 | 1.06 |
| Polar solvent | Dimethyl sulfoxide | 140 | 80 | 1.75 |
| | Dimethylformamide | 135 | 60 | 2.25 |
| | Dimethylacetamide | 135 | 80 | 1.69 |

The above-described organic solvents can be mixed with water and used for a reaction in the form of a biphasic solvent. Examples of the biphasic solvent of a water-nonmiscible organic solvent and water that is used in the present embodiment include solvents in which water and an organic solvent form two layers, solvents in which an organic solvent is dispersed in a water solvent, and solvents in which water is dispersed in an organic solvent. In the present invention, a biphasic solvent formed of a combination of water and a water-nonmiscible organic solvent is a preferred mode since it reduces the load at the time of solvent recovery.

A mass ratio of the organic solvent and water is not particularly limited; however, it is usually 1:1 to 4:1, preferably 2:1 to 4:1. When the mass ratio is in this range, 5-HMF can be efficiently extracted from an aqueous phase, and deterioration of 5-HMF can be reduced, which is preferred.

The above-described mass ratio represents a mass ratio at 25°C under atmospheric pressure.

<Isomerization Catalyst>

The isomerization catalyst used in the present embodiment is a catalyst that isomerizes glucose into fructose. The isomerization catalyst is preferably a metal oxide, and examples thereof include: metal oxides and metal composite oxides, such as aluminum oxide, magnesium oxide, calcium oxide, zirconium oxide, $Nb_2O_5$, $YNbO_4$, $Ta_2O_5$, $Nb_2O_5$-$WO_3$, $Nb_2O_5$-$MoO_3$, $TiO_2$-$WO_3$, $TiO_2$-$MoO_3$, $SiO_2$-$Nb_2O_5$, and $SiO_2$-$Ta_2O_5$; and immobilization catalysts such as clay. Thereamong, the isomerization catalyst is preferably aluminum oxide, magnesium oxide, calcium oxide, or zirconium oxide, particularly preferably aluminum oxide.

These isomerization catalysts may be used singly, or in any combination of two or more thereof at any ratio.

When aluminum oxide is used as the isomerization catalyst, the specific surface area of aluminum oxide is preferably 100 $m^2/g$ or more, more preferably 150 $m^2/g$ or more. An upper limit thereof is not particularly limited; however, it is usually 500 $m^2/g$ or less, preferably 300 $m^2/g$ or less. When the specific surface area of aluminum oxide is in this range, the probability of aluminum oxide to come into contact with glucose is sufficiently high, so that the isomerization rate can be improved.

The specific surface area of aluminum can be measured by a transmission method or a gas adsorption method. It is noted here that the value of the specific surface area is the same regardless of whether it is measured by a transmission method or a gas adsorption method.

<Dehydration Catalyst>

The dehydration catalyst used in the present embodiment catalyzes intramolecular dehydration reactions of glucose and fructose. The dehydration catalyst is preferably a solid acid. The term "solid acid" used herein refers to a solid that chemically adsorbs a base, and either gives a proton to or receives an electron pair from a reactant. The solid acid is preferably one which is solid at 25°C and exhibits acidity.

Specific examples of the solid acid include heteropoly acid, phosphoric acid-diatomaceous earth, activated carbon, steam-activated carbon, phosphoric acid-activated carbon, acid-modified silica gel, acid-modified silica alumina, zeolite, ion exchange resins, sulfuric acid immobilization catalysts such as sulfated $ZrO_2$, and phosphoric acid immobilization catalysts such as phosphoric acid-treated titania and niobia. Thereamong, an ion exchange resin is preferred. The ion exchange resin is not particularly limited as long as it has a Bronsted acid center on the surface of a carrier, and examples of such an ion exchange resin include those having a carboxyl group and/or a sulfonate group. From the standpoint of reactivity, a sulfonate group-containing strongly acidic cation exchange resin is preferred. From the standpoint of catalyst recovery, an insoluble catalyst (also referred to as "heterogeneous catalyst") is preferred rather than a water-soluble catalyst.

These dehydration catalysts may be used singly, or in any combination of two or more thereof at any ratio.

The specific surface area of the dehydration catalyst is preferably 40 $m^2/g$ or more, more preferably 50 $m^2/g$ or more. An upper limit thereof is not particularly limited; however, it is usually 200 $m^2/g$ or less, preferably 100 $m^2/g$ or less. When the specific surface area of the dehydration catalyst is in this range, the probability of the dehydration catalyst to come into contact with fructose generated from glucose is sufficiently high, so that the efficiency of dehydration reaction can be improved.

The specific surface area of the dehydration catalyst can be measured by a transmission method or a gas adsorption method.

<Mixed Catalyst>

The above-described isomerization catalyst and the above-described dehydration catalyst are, in the form of a mixed catalyst, brought into contact with glucose that is a raw material.

A specific mode of bringing the mixed catalyst into contact with glucose is not particularly limited. A mixed catalyst obtained by substantially uniformly mixing the isomerization catalyst and the dehydration catalyst may be brought into contact with glucose, or a catalyst obtained by alternately disposing the isomerization catalyst and the dehydration catalyst in layers may be filled into a column, and a solution containing an organic solvent and glucose may be passed through this column and thereby brought into contact with the catalyst. For the passing of the solution through the column, an embodiment in which a liquid line for organic solvent and a liquid line for glucose solution are separately prepared and these lines are merged prior to the contact with the catalyst may be employed.

Alternatively, a biphasic solvent, a mixed catalyst, and glucose may be put into a reactor and allowed to react with stirring.

By using a mixed catalyst, isomerization reaction and dehydration reaction are made more likely to proceed as compared to bringing the individual catalysts into contact with the raw material. Further, this method is advantageous in that it uses only one reaction tank and can thus shorten the reaction step as compared to a method of sequentially bringing the raw material into contact with an isomerization catalyst tank and a dehydration catalyst tank.

In the present embodiment, a mixing ratio of the isomerization catalyst and the dehydration catalyst is not particularly limited, and it is determined with consideration of the specific surface area of each catalyst. The mixing ratio is usually 1:3 to 3:1, preferably 1:3 to 1:2, in terms of mass ratio. When the mixing ratio is in this range, fructose partially generated by the isomerization reaction of glucose can be promptly dehydrated.

A ratio between a value obtained by multiplying the specific surface area and the amount of the isomerization catalyst and a value obtained by multiplying the specific surface area and the amount of the dehydration catalyst is not particularly limited. This ratio is preferably 1:20 to 20:1, more preferably 1:10 to 10:1, particularly preferably 1:1 to 10:1. When the ratio is in this range, the isomerization reaction of glucose, which is likely to be rate-limiting, can be accelerated, so that partially generated fructose can be promptly dehydrated.

The amount of the mixed catalyst used in the present embodiment is not particularly limited. It is usually 0.01 g or more, preferably 0.1 g or more, more preferably 0.5 g or more, but usually 10 g of less, preferably 5 g or less, more preferably 3 g or less, with respect to 1 g of glucose. When the catalyst amount is equal to or greater than the above-described lower limit, reactions can proceed efficiently. Meanwhile, when the catalyst amount is equal to or less than the above-described upper limit, the cost can be cut down by a reduction in excess catalyst.

<Glucose>

The method of producing 5-HMF according to the present embodiment is characterized by allowing the above-described mixed catalyst and glucose to come into contact and react with each other. As a supply source of glucose, glucose itself (monosaccharide) may be used, or a sugar polymer (an oligosaccharide or a polysaccharide) containing glucose as a constituent sugar may be used. Further, a glucose derivative may be used as the supply source of glucose.

The sugar polymer containing glucose as a constituent sugar is not particularly limited. Examples thereof include sucrose, maltose, trehalose, turanose, isomaltulose, cellobiose, isomaltose, nigerose, maltulose, isomaltulose, gentiobiose, maltotriose, 1-kestose, maltooligosaccharide, dextrin, dextran, starch, cellulose, lactose, lactulose, mannan, and xyloglucan. Thereamong, sucrose and starch are preferred. When a sugar polymer containing glucose as a constituent sugar is used as the supply source of glucose, glucose can be supplied while decomposing the sugar polymer by a heating reaction, and glucose can be concurrently converted into 5-HMF through an isomerization reaction and an intramolecular dehydration reaction using the mixed catalyst.

The glucose derivative is not particularly limited as long as it has a functional group subjected to the dehydration reaction, such as a hydroxy group, and examples thereof include modified sugars, such as amino sugar, etherified sugar, halogenated sugar and phosphorylated sugar. Examples of such derivatives include glucosamine and glucose-6-phosphate. These derivatives are subjected to a pretreatment such as hydroxylation and reduction treatment, and can be converted into 5-HMF through an isomerization reaction and an intramolecular dehydration reaction using the mixed catalyst, while supplying glucose.

Further, the supply source of glucose may contain a hexose other than glucose as a sugar polymer or a monosaccharide. The hexose to be contained in addition to glucose is not particularly limited; however, fructose is preferred because of its reactivity, availability, cost, and the like.

The supply source of glucose contains glucose in an amount of preferably not less than 10% by mass, more preferably not less than 50% by mass, with respect to the whole sugar and, from the standpoint of raw material cost and availability, the supply source of glucose particularly preferably contains not less than 90% by mass of glucose. According to the production method of the present embodiment, 5-HMF can be obtained with a high yield and a high selectivity even when a raw material containing glucose in the above-described range is used; therefore, the production cost can be kept low as compared to a case of using fructose as a raw material.

In the present embodiment, the glucose content in a glucose solution obtained by dissolving the supply source of glucose in the above-described biphasic solvent is preferably 1% by mass to 90% by mass, more preferably 3% by mass to 80% by mass, particularly preferably 5% by mass to 70% by mass, with respect to the amount of water. The glucose solution may also contain a solid component formed of a sugar that is left undissolved. The solid concentration of the solution is preferably 1% by mass to 85% by mass, more preferably 5% by mass to 80% by mass, particularly preferably 20% by mass to 75% by mass. The use of a solution having a solid concentration of a certain level or lower can further inhibit the generation of a sugar condensate as a by-product, while the use of a solution having a solid concentration of a certain level or higher allows the reactions to proceed efficiently.

The conditions for bringing the mixed catalyst and glucose into contact are not particularly limited. The reaction temperature is preferably 80°C or higher, more preferably 100°C or higher, but preferably 200°C or lower, more preferably 150°C or lower. When the reaction temperature is equal to or higher than the above-described lower limit, the reactions are allowed to proceed efficiently. Meanwhile, when the reaction temperature is equal to or lower than the above-described upper limit, the generation of a by-product can be suppressed, and the deterioration of the catalyst can be inhibited. The reaction time is not particularly limited; however, it is preferably 1 to 24 hours, more preferably 2 to 10 hours. The reaction time can be set as appropriate in accordance with the reaction temperature and, for example, when the reaction temperature is high, the reaction rate is increased, and the reaction time can thus be shortened.

The pressure during the reactions is not particularly limited; however, it is preferably 0.01 MPa or higher, more preferably 0.1 MPa or higher, but preferably 10 MPa or lower, more preferably 1 MPa or lower. When this pressure is in the above-described range, the generation of a by-product can be inhibited.

<Alkali Metal Salt or Alkaline Earth Metal Salt>

In the present embodiment, the above-described biphasic solvent preferably contains an alkali metal salt or an alkaline earth metal salt in an amount of 80 parts by mass or less with respect to 100 parts by mass of glucose. This amount is usually 80 parts by mass or less, more preferably 50 parts by mass or less. A lower limit thereof is not particularly limited; however, it is usually not less than 1 part by mass, more preferably not less than 0.01 parts by mass. It is noted here that the biphasic solvent does not have to contain an alkali metal salt or an alkaline earth metal salt.

Incorporation of the above-described amount of an alkali metal salt or an alkaline earth metal salt into the biphasic solvent may facilitate the below-described oil-water separation and is thus preferred.

The alkali metal salt or the alkaline earth metal salt is not particularly limited, and any known alkali metal salt

or alkaline earth metal salt can be used. Specific examples thereof include sodium chloride and potassium chloride.

<Oil-Water Separation>

The production method of the present embodiment may further include the oil-water separation step. The oil-water separation step is the step of separating an organic solvent phase and an aqueous phase, which is performed for the purpose of separating a target compound 5-HMF and other compounds from a reaction solution obtained by the above-described reactions. Specifically, the organic solvent phase contains an oil-soluble component mainly composed of 5-HMF generated by the above-described reactions, and the aqueous phase contains water-soluble components such as sugar raw materials and catalysts. An oil-water separation method is not particularly limited as long as it is a method of separating the organic solvent phase and the aqueous phase based on their difference in specific gravity. For example, a method of putting the reaction solution into a separation tank and leaving the reaction solution to stand until the organic solvent phase and the aqueous phase are separated may be employed. A specific duration of leaving the reaction solution to stand is not particularly limited; however, it is usually 0.1 hours or longer, preferably 1 hour or longer, but usually 48 hours or shorter, preferably 24 hours or shorter.

In the case of performing the oil-water separation, the temperature thereof is preferably room temperature to 90°C. A lower limit of this temperature is more preferably 30°C, and an upper limit is more preferably 60°C, still more preferably 40°C. By controlling the reaction temperature to be in this range, the extraction efficiency at the time of recovery is improved, so that the operating time can be shortened.

In the production method of the present embodiment, since reactions are performed in a biphasic solvent of a water-nonmiscible organic solvent and water, the time required for the oil-water separation after the reactions can be shortened. By this, the resulting 5-HMF is separated from the catalysts; therefore, deterioration of 5-HMF is inhibited, so that 5-HMF can be produced with a yield higher than that of a conventional method. This 5-HMF can be further separated and purified by removing the organic solvent by distillation from the organic solvent phase recovered by the oil-water separation. For the purification, a treatment such as centrifugation, drying, or extraction may be performed. In this process, the distilled organic solvent may be reused.

In the oil-water separation, only the organic solvent phase may be recovered, and the aqueous phase may be discarded. Further, the production method of the present embodiment may include the step of recovering the aqueous phase. The aqueous phase contains water-soluble components such as sugar raw materials and catalysts. Examples of sugars contained in the aqueous phase include those sugars that are exemplified above for the supply source of glucose and the above-described hexose other than glucose, as well as sugars generated by isomerization reactions of the above-exemplified sugars and/or a sugar condensation reaction that is a side reaction. Specific examples include glucose and fructose. The aqueous phase may be recovered and, after an addition of a raw material sugar to the thus recovered aqueous phase, this aqueous phase may be introduced again to a 5-HMF production reactor. This process is preferred from the standpoint of production specific consumption since it enables to efficiently use an unreacted raw material sugar and to use recovered catalysts as is.

The thus recovered sugar and mixed catalyst may be further purified by a treatment such as centrifugation, drying, or extraction. The recovered sugar and mixed catalyst can be reused for the 5-HMF generation reactions.

The 5-HMF obtained by the production method of the present embodiment can be, after being converted into a derivative as required, utilized as a raw material of a biofuel, a synthetic resin, a flavor-imparting component, a physiologically active component, a food product, a pharmaceutical product, or the like. Examples of the derivative include 2,5-dimethylfuran (DMF), 2,5-furandicarboxylic acid (FDCA), and 5-methoxymethyl furfural. As a method of producing these derivatives, any known method can be employed.

The method of the present embodiment may also be employed for the production of a compound that is generated from a hexose other than glucose through isomerization and dehydration reactions. As for catalysts, reaction conditions, and the like to be used, those described above can be applied.

<Second Gist>

Another embodiment (second gist) of the present invention is a method of producing a furan derivative having an acetal group and a carbonyl group, which method includes the first oxidation step of bringing HMF acetal into contact with an oxidation catalyst.

Methods of producing FDCA or a furandicarboxylic acid ester from HMF are disclosed in Patent Documents 3 and 4 and the like; however, the present inventors discovered that FDCA or a furandicarboxylic acid ester can be produced from HMF with a high yield by a production method that goes through a furan derivative having an acetal group and a carbonyl group. In the case of going through the above-described furan derivative, the method of producing FDCA or a furandicarboxylic acid ester from HMF may include the following steps:

- the acetalization step of acetalizing HMF;
- the deprotection step of deprotecting the furan derivative having an acetal group and a carbonyl group;
- the recovery step of recovering a diol from a reaction solution after the deprotection step; and
- the second oxidation step of oxidizing a compound obtained in the deprotection step.

These steps will now be described one by one.

<Acetalization Step>

In the acetalization step, HMF is acetalized. HMF can be derived from a biomass raw material. For example, HMF can be obtained by, but not limited to, a known method disclosed in Green Chemistry 16 (2014), 4816-4838, or the like. In one example, a sugar solution containing oligosaccharides, disaccharides, and monosaccharides is obtained by decomposition (saccharification) of polysaccharides contained in a biomass raw material into saccharides that are structural units of the polysaccharides through a known pretreatment/saccharification step such as a chemical treatment of the biomass raw material with an acid, an alkali, or the like, a biological treatment using a microorganism, or a physical treatment, and HMF can be obtained from this sugar solution.

The biomass raw material encompasses those materials in which the solar light energy is converted and stored into the form of starch, cellulose, hemicellulose, or the like by plant photosynthetic activity, and products obtained by processing plant bodies. Examples of the biomass raw material include wood, rice straw, chaff, rice bran, long-stored rice, corn, sugarcane, cassava, sago palm, cardoon, switchgrass, pine wood, poplar wood, maple wood, soy pulp, corn cob, corn stover, corn fiber, tapioca, bagasse, vegetable oil refuse, potatoes, buckwheat, soybean, wastepaper, papermaking residues, aquatic product residues, livestock excreta, sewage sludge, and food wastes. Thereamong, plant resources such as wood, rice straw, chaff, rice bran, long-stored rice, corn, sugarcane, cassava, sago palm, cardoon, switchgrass, pine wood, poplar wood, maple wood, soy pulp, corn cob, corn stover, corn fiber, tapioca, bagasse, vegetable oil refuse, potatoes, buckwheat, soybean, wastepaper, papermaking residues, and food wastes are preferred; wood, rice straw, chaff, long-stored rice, corn, sugarcane, cassava, sago palm, cardoon, switchgrass, pine wood, poplar wood, maple wood, corn cob, corn stover, corn fiber, bagasse, potatoes, wastepaper, papermaking residues, and food wastes are more preferred; and corn, sugarcane, cassava, sago palm, bagasse, cardoon, switchgrass, pine wood, poplar wood, maple wood, corn cob, corn stover, corn fiber, wastepaper, papermaking residues, and food wastes are most preferred.

As saccharides derived from the above-exemplified biomass raw materials, hexoses such as glucose, mannose, galactose, fructose, sorbose, and tagatose, and disaccharides/oligosaccharides/polysaccharides such as saccharose, starch, cellulose, and hemicellulose are usually used and, thereamong, glucose and fructose are preferred because of their high reaction yields. Meanwhile, as saccharides derived from plant resources in a broader sense, cellulose and hemicellulose are preferred.

Acetalization is usually allowed to proceed in a solvent. A catalyst may be incorporated into the solvent and, from the standpoint of allowing the reaction to proceed in a short time, it is preferred to incorporate an insoluble solid catalyst into the solvent and to perform the reaction in a heterogeneous system. Examples of a preferred catalyst include: acidic cation exchange resins, such as AMBERLYST, AMBERLITE, and DIAION; metal oxides and metal composite oxides, such as zeolite, $Nb_2O_5$, $YNbO_4$, $Ta_2O_5$, $Nb_2O_5$-$WO_3$, $Nb_2O_5$-$MoO_3$, $TiO_2$-$WO_3$, $TiO_2$-$M_OO_3$, $SiO_2$-$Nb_2O_5$, and $SiO_2$-$Ta_2O_5$; sulfuric acid immobilization catalysts, such as clay and sulfated $ZrO_2$; and phosphoric acid immobilization catalysts, such as phosphoric acid-treated titania and niobia. Thereamong, for the reason of selectively allowing only the acetalization reaction of HMF to proceed, acidic cation exchange resins, such as AMBERLYST, AMBERLITE, and DIAION, as well as zeolite, $Nb_2O_5$, $Nb_2O_5$-$WO_3$, and $Nb_2O_5$-$MoO_3$ are more preferred, and acidic cation exchange resins such as AMBERLYST, AMBERLITE, and DIAION are particularly preferred.

These catalysts may be used singly, or in combination of two or more thereof. When two or more catalysts are used, a combination thereof is not particularly limited, and the catalysts to be combined may each be a co-catalyst whose metal has a catalytic activity, or a promotor that improves the catalytic activity of other metal.

As the solvent, water or an organic solvent is usually used; however, there are cases where it is preferred to use a mixed solvent obtained by combining water and an organic solvent. From the standpoint of cost advantage, it is preferred to use a single solvent as the reaction solvent.

The organic solvent to be used is not particularly limited, and examples thereof include: ethers having 4 to 20 carbon atoms, such as diethyl ether, tetrahydrofuran (THF), tetrahydropyran, methyltetrahydropyran, dipropyl ether, diisopropyl ether, methyl-tert-butyl ether, butyl ether, pentyl ether, hexyl ether, octyl ether, nonyl ether, decyl ether, ethylene glycol dimethyl ether, ethylene glycol diethyl ether, diethylene glycol diethyl ether, and triethylene glycol dimethyl ether; ketones having 4 to 20 carbon atoms, such as 2-butanone (methyl ethyl ketone), 2-pentanone, 3-pentanone, 2-hexanone, 3-hexanone, cyclohexanone, 4-methyl-2-pentanone (methyl isobutyl ketone), 2-heptanone, 5-methyl-2-hexanone, 2,4-dimethyl pentanone, 5-nonanone, 4-decanone, 5-decanone, 2-undecanone, 4-undecanone, 3-dodecanone, 2-tridecanone, 2-tetradecanone, 4-tetradecanone, 2-pentadecanone, 3-pentadecanone, 7-pentadecanone, 2-hexadecanone, 3-hexadecanone, 4-hexadecanone, 6-hexadecanone, 2-heptadecanone, 4-heptadecanone, 9-heptadecanone, 3-octadecanone, and acetophenone; esters, such as ethyl acetate, propyl acetate, and butyl acetate; monoalcohols having 1 to 20 carbon atoms, such as methanol, ethanol, 1-propanol, 2-propanol, 1-butanol, 2-butanol, 2-methyl-1-propanol, 2-methyl-2-propanol, 1-pentanol, 2-pentanol, 3-pentanol, 3-methyl-1-butanol, 2,2-dimethyl-1-propanol, 1-hexanol, 2-hexanol, 3-hexanol, 2-methyl-1-pentanol, 1-heptanol, 2-heptanol, 1-octanol, 2-octanol, 3-octanol, 2-ethyl-1-hexanol, 1-nonanol, 1-decanol, 1-undecyl alcohol, 1-lauryl alcohol, 1-tridecyl alcohol, 1-tetradecanol, 1-pentadecyl alcohol, 1-hexadecanol, cis-9-hexadecen-1-ol, 1-heptadecanol, 1-octadecanol, 16-methylheptadecen-1-ol, nonadecyl alcohol, and arachidyl alcohol; diols having 1 to 6 carbon atoms, such as ethylene glycol, 1,3-propanediol, 2-methyl-1,3-propanediol, 1,3-butanediol, 1,3-pentanediol, 1,2-propanediol, 1,2-butanediol, and 1,2-pentanediol; phenols having 6 to 12 carbon atoms, such as phenol, methyl phenol, ethyl phenol, propyl phenol, and butyl phenol; saturated aliphatic hydrocarbon compounds having 3 to 12 carbon atoms, such as pentane, hexane, cyclohexane, heptane, octane, nonane, decane, dodecane, and isododecane; aromatic hydrocarbons, such as toluene, xylene, trimethylbenzene, 1,2,3,4-tetrahydronaphthalene, and 1-methylnaphthalene; lactones, such as γ-butyrolactone and γ-valerolactone; halogenated hydrocarbons, such as dichloromethane, chloroform, dichloroethane, tetrachloroethane, and hexachloroethane; and dimethylacetamide, *N*,*N*-dimethylformamide, *N*-methylmorpholine, *N*-methyl-2-pyrrolidinone, 1,3-dimethyl-2-imidazolidinone, hexamethylphosphoric triamide, tetramethyl urea, dimethyl sulfoxide, sulfolane, isosorbide, isosorbide dimethyl ether, propylene carbonate, and mixtures thereof. In addition, for example, an ionic liquid of a pyrrolidinium salt such as 1-butyl-1-methylpyrrolidinium bromide, a piperidinium salt such as 1-butyl-1-methylpiperidinium triflate, a pyridinium salt such as 1-butylpyridinium tetrafluoroborate, an imidazolium salt such as 1-ethyl-3-methylimidazolium chloride, an ammonium salt such as tetrabutylammonium chloride, a phosphonium salt such as tetrabutylphosphonium bromide, a sulfonium salt such as triethylsulfonium bis(trifluoromethanesulfonyl)imide, or the like may be used as well.

Thereamong, the organic solvent is preferably at least one selected from the group consisting of ethers, ketones, diols, esters, lactones, aromatic hydrocarbons, halogenated hydrocarbons, and saturated aliphatic hydrocarbons, more preferably at least one selected from the group consisting of ethers, ketones, diols, aromatic hydrocarbons, and saturated aliphatic hydrocarbons, still more preferably at least one selected from the group consisting of ethers, ketones, aromatic hydrocarbons, and saturated aliphatic hydrocarbons. Specifically, the organic solvent is preferably methyltetrahydropyran, 2-butanone (methyl ethyl ketone), 4-methyl-2-pentanone (methyl isobutyl ketone), or toluene.

In the acetalization step, a diol is usually added for the acetalization of HMF, and this diol may be any alcohol having two or more hydroxyl groups, and may be a trihydric alcohol such as glycerol, or a tetrahydric alcohol such as erythritol. Further, catechol is also preferably used.

The diol to be added is determined as appropriate in accordance with the type of the acetal intermediate to be generated, and is preferably a diol having 2 to 10 carbon atoms, examples of which include ethylene glycol, 1,3-propanediol, 2-methyl-1,3-propanediol, neopentyl glycol, 1,3-butanediol, 1,3-pentanediol, 1-phenyl-1,3-propanediol, 2-phenyl-1,3-propanediol, 1,2-propanediol, 1,2-butanediol, 1,2-pentanediol, and 1-phenyl-ethylene glycol. Among these diols, a diol that forms a 6-membered ring structure is preferred since such a diol improves the stability of the resulting acetal intermediate, and specific examples of such a diol include 1,3-propanediol, 2-methyl-1,3-propanediol, neopentyl glycol, 1,3-butanediol, 1,3-pentanediol, 1-phenyl-1,3-propanediol, and 2-phenyl-1,3-propanediol. It is possible to use two or more kinds of diols; however, a single kind of diol is usually used.

It is noted here that a diol may be used as the above-described organic solvent. When a diol is used as the organic solvent, the diol is preferably in a liquid state at normal temperature. In this case, the acetalization of HMF can be performed without separately adding a diol to the organic solvent. When a diol is used as the organic solvent, specific examples of the diol include ethylene glycol, 1,3-propanediol, 2-methyl-1,3-propanediol, 1,3-butanediol, 1,3-pentanediol, 1,2-propanediol, 1,2-butanediol, and 1,2-pentanediol.

The reaction in the acetalization step can be performed by, for example, a batch method or a flow method. As for a liquid-contact material of a reactor, its flow channels, and the like, the material of the reactor is not particularly limited to, for example, glass, stainless steel (SUS), iron, or other metal; however, a reactor made of glass or stainless steel (SUS) is preferably used because of its corrosion resistance and high energy transfer efficiency. Further, from the standpoint of the cost of industrial production equipment, it is preferred to use a reactor made of stainless steel (SUS).

Moreover, in the acetalization step, a membrane may be installed in the reaction system, and the acetalization reaction may be performed while dehydrating the inside of the reaction system with utilization of reaction separation. The membrane to be installed in the reaction system is not limited, and examples thereof include a zeolite membrane, a silica membrane, and a carbon membrane. For selective discharge of water, it is preferred to use a hydrophilic zeolite membrane that has a certain pore size conforming to the size of water molecules. Examples of the type of the zeolite membrane include FAU-type, BEA-type, MOR-type, MFI-type, FER-type, LTA-type, CHA-type, and DDR-type, and a zeolite membrane of LTA-type (pore size = about 4 Å), CHA-type (pore size = about 3.8 Å), or DDR-type (pore size = about 3.2 Å), which has a pore size similar to the size of water molecules (about 2.7 Å) is preferably used for dehydration. The silica ($SiO_2$)/alumina ($Al_2O_3$) ratio of the zeolite membrane is in a range of preferably 2 to 40, more preferably 2 to 20. It is preferred to select a membrane having a silica/alumina ratio in this range since such a membrane is hydrophilic

and has good membrane stability (acid resistance) and high dehydration capacity.

The reaction temperature in the acetalization step is preferably room temperature to 120°C. A lower limit of the reaction temperature is more preferably 30°C, still more preferably 40°C, and an upper limit is more preferably 100°C, still more preferably 80°C. By controlling the reaction temperature to be in this range, an increase in the reaction time, concurrence of a side reaction, reaction runaway, coloring of the resulting product, and the like can be inhibited.

The reaction time in the acetalization step is usually 1 minute to 30 hours, preferably 10 minutes to 20 hours, more preferably 30 minutes to 15 hours, taking the point when stirring is initiated as the starting point in the case of a batch method. By controlling the reaction time to be in this range, concurrence of a side reaction, coloring of the resulting product, and the like can be inhibited. It is noted here that the reaction time is not limited to this in the case of employing a flow method.

The reaction pressure in the acetalization step is usually, but not particularly limited to, normal pressure.

<First Oxidation Step>

In the first oxidation step, a furan derivative having an acetal group and a carbonyl group is synthesized by oxidation of HMF acetal. It is noted here that the oxidation in this step includes oxidative esterification.

In the present embodiment, HMF acetal and an oxidation catalyst are brought into contact with each other in the first oxidation step to synthesize a furan derivative having an acetal group and a carbonyl group. This furan derivative is preferably a compound represented by the following Formula (1), more preferably a compound represented by the below-described Formula (2), still more preferably a compound represented by the below-described Formula (4).

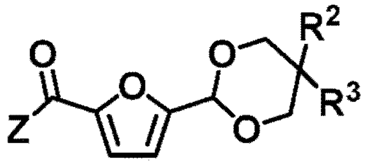 (1)

In Formula (1), Z represents a hydrogen atom or a hydroxyl group, and $R^2$ and $R^3$ are independently selected from a hydrogen atom, an alkyl group having 1 to 6 carbon atoms, and an aryl group having 6 to 12 carbon atoms that optionally has an alkyl substituent.

Examples of the alkyl substituent include alkyl groups having 1 to 6 carbon atoms.

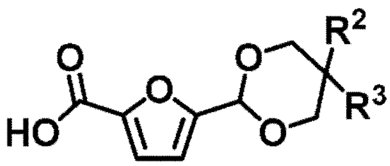 (2)

In Formula (2), $R^2$ and $R^3$ are independently selected from a hydrogen atom, an alkyl group having 1 to 6 carbon atoms, and an aryl group having 6 to 12 carbon atoms that optionally has an alkyl substituent.

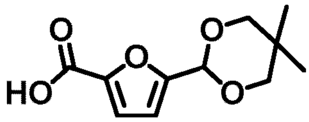 (4)

The oxidation of HMF acetal is performed in the presence of a catalyst, and examples of this catalyst include: the metals of the periodic table Groups 8 to 10, such as platinum, palladium, nickel, iron, ruthenium, and cobalt, and compounds thereof; the metals of the periodic table Group 11, such as copper, silver, and gold, and compounds thereof; the metals of the periodic table Group 12, such as zinc, and compounds thereof; the metals of the periodic table Group 13, such as indium, and compounds thereof; the metals of the periodic table Group 14, such as tin and lead, and compounds thereof; the metals of the periodic table Group 7, such as manganese and rhenium, and compounds thereof; the metals of the periodic table Group 6, such as chromium and molybdenum, and compounds thereof; the metals of the periodic table Group 5, such as niobium and tantalum, and compounds thereof; the metals of the periodic table Group 4, such as zirconium, and compounds thereof; the metals of the periodic table Group 3, such as scandium, and compounds thereof; the metals of the periodic table Group 2, such as magnesium and calcium, and compounds thereof;

the metals of the periodic table Group 1, such as cesium, and compounds thereof; and mixtures (including alloys) of these metals. Particularly, the metals of the periodic table Groups 6 to 12 as well as compounds and mixtures (including alloys) thereof are preferred, and the metals of the periodic table Groups 7 to 11 and compounds thereof are more preferred. Thereamong, platinum, palladium, rhenium, magnesium, copper, silver, gold, zinc, manganese, cobalt, and compounds thereof are particularly preferred and, for example, platinum, palladium, rhenium, copper, silver, gold, cobalt, manganese, and compounds thereof can be most preferably used. Examples of the compounds include oxides, alkoxy compounds, alkyl compounds, and organic acid salts of the above-described metals, and the catalyst is preferably a metal, a metal oxide, or a metal organic acid salt. These metals and their compounds may be mixed, and the catalyst may be, for example, a combination of a cobalt compound, a manganese compound, and a bromine compound.

Further, these metal catalysts are preferably used as metal-supported catalysts supported on various carriers. Examples of the carriers include natural minerals, such as carbon, silica, alumina, titania, zirconia, niobia, ceria, tungsten oxide, silicon carbide, boron carbide, titanium carbide, diatomaceous earth, and layered silicates. Thereamong, carbon, titania, and ceria are preferred in terms of the ease of preparation and the activity of catalyst. These catalysts may be used singly or simultaneously in combination of two or more thereof, or may be added afterwards.

In this step, in the case of performing oxidation reaction of HMF acetal in the presence of a metal catalyst, it can be performed by, for example, a batch method or a flow method, and it is preferred to employ a flow method because of its high production efficiency.

In the case of performing the oxidation reaction by a batch method, the amount of the above-described catalyst to be used is preferably 0.002 ppm by mass to 100,000 ppm by mass in terms of the amount of metal with respect to a raw material. A lower limit of the amount of the catalyst to be used is more preferably 0.02 ppm by mass, still more preferably 0.2 ppm by mass, particularly preferably 2 ppm by mass, in terms of the amount of metal with respect to a raw material. Further, an upper limit of the amount of the catalyst to be used is more preferably 50,000 ppm by mass, still more preferably 20,000 ppm by mass, particularly preferably 10,000 ppm by mass, in terms of the amount of metal with respect to a raw material. By using the catalyst in such an amount, not only the above-described furan derivative can be efficiently produced with a moderate reaction time, but also an increase in the cost of the catalyst and coloring of the resulting product can be inhibited. It is noted here that the amount of the catalyst to be used is not limited to this in the case of employing a flow method.

In this step, the presence of a base enables not only to improve the stability of the acetal and inhibit a side reaction, but also to improve the yield of a target product in some cases; therefore, the oxidation reaction may be performed in the presence of a base. Examples of this base include: inorganic bases, such as sodium hydroxide, sodium carbonate, sodium bicarbonate, potassium hydroxide, potassium carbonate, and potassium bicarbonate; and organic bases such as triethylamine, among which an inorganic base is preferred because of the efficiency of its removal from the product.

In the case of performing the oxidation reaction in the presence of a base, the amount of the base to be used is not particularly limited; however, a lower limit thereof is 0.01 equivalents or more, preferably 0.1 equivalents or more, while an upper limit thereof is 10 equivalents or less, preferably 5 equivalents or less, more preferably 3 equivalents or less, with respect to the amount of HMF acetal. By using the base in such an amount, not only the stability of HMF acetal can be improved and a side reaction can be inhibited, but also the yield of a target product can be improved.

The reaction temperature in this step is preferably room temperature to 200°C. A lower limit of the reaction temperature is more preferably 30°C, still more preferably 40°C, particularly preferably 60°C, and an upper limit is more preferably 180°C, still more preferably 150°C, particularly preferably 130°C, most preferably 80°C. By controlling the reaction temperature to be in this range, an increase in the reaction time, concurrence of a side reaction, decomposition of the diol used as an acetalization agent, reaction runaway, coloring of the resulting product, and the like can be inhibited.

An oxygen source used for the oxidation reaction is not particularly limited to an oxygen-containing organic compound, an inorganic peroxide, an oxygen-containing gas, or the lie; however, usually, an oxygen-containing gas that does not require separation and purification after the completion of the reaction, such as oxygen gas or air, is used. The oxidation reaction can be performed by confining an oxygen-containing gas in a reactor in a batch method, or by circulating an oxygen-containing gas in a reactor in a flow method, and it is preferred to perform the oxidation reaction by a flow method.

In the case of using an oxygen-containing gas, the oxygen content therein is usually 20% by volume or more, and may be 50% by volume or more. Alternatively, pure oxygen (99% or more) may be used. From the standpoint of reducing the risk of explosion to ensure the reaction safety, it is preferred to use the air that contains about 21% by volume of oxygen, or an oxygen-containing gas having substantially the same composition as the air. By using such an oxygen-containing gas, not only the reaction selectivity can be improved, but also the oxidative decomposition reaction of the diol required for obtaining a cyclic acetal of HMF can be inhibited.

The reaction time in this step is not particularly limited in the case of performing the oxidation reaction by a flow method. In the case of performing the oxidation reaction by a batch method, the reaction time is usually 10 minutes to 30 hours, preferably 30 minutes to 20 hours, more preferably 1 hour to 10 hours, still more preferably 1 hour to 5 hours,

taking the point when the oxygen-containing gas is initially introduced into the reactor as the starting point. By controlling the reaction time to be in this range, concurrence of a side reaction and reaction runaway can be inhibited, and the production cost can be reduced.

In the case of performing the oxidation reaction by a flow method, the rate at which the oxygen-containing gas is circulated in the reactor is usually 1 mL/min to 100 L/min, preferably 10 mL/min to 10 L/min, more preferably 100 mL/min to 5 L/min, still more preferably 500 mL/min to 1 L/min. By controlling the above-described rate to be in this range, not only the oxygen consumption efficiency and the production efficiency of a target product can be improved, but also concurrence of a side reaction and reaction runaway can be inhibited, and the production cost can be reduced.

Examples of a method of circulating the oxygen-containing gas in the reactor include a method of blowing the oxygen-containing gas into a liquid layer of the reactor, and a method of blowing the oxygen-containing gas into a gaseous layer portion of the reactor; however, the former method is preferred.

The oxygen-containing gas may be circulated continuously or intermittently; however, a method of continuously circulating the oxygen-containing gas is preferred. In the case of intermittently circulating the oxygen-containing gas, it is preferred to appropriately adjust the intervals of the intermittent circulation such that shortage of oxygen is prevented.

Further, the oxidation reaction may be performed by supplying the oxygen-containing gas such that the inside of the reaction vessel is pressurized. In this case, the reaction pressure is usually 0.1 MPa or higher, preferably 0.3 MPa or higher, more preferably 0.4 MPa or higher, but usually 15 MPa or lower, preferably 10 MPa or lower, more preferably 5 MPa or lower, still more preferably 3 MPa or lower, particularly preferably 1 MPa or lower. By controlling the reaction pressure to be in this range, a high-pressure reaction equipment is no longer necessary, and concurrence of a side reaction and the oxidative decomposition reaction of the diol required for obtaining a cyclic acetal of HMF can be inhibited.

The reaction solvent used in this step is not particularly limited; however, it is preferably a solvent containing water that dissolves HMF acetal used as a raw material. In this case, a mixed solvent of the reaction solvent used in the above-described acetalization step and water, a mixed solvent of the above-described diol and water, or water alone can be used. When a cyclic acetal intermediate is isolated and then oxidized, the solvent does not necessarily have to be a mixed solvent, and a water solvent may be used. In the case of using a mixed solvent with water, the content of water therein is not particularly limited as long as it is 1 equivalent or more with respect to the cyclic acetal intermediate; however, a lower limit thereof is, in terms of volume ratio, 1% by volume or more, preferably 10% by volume or more, more preferably 50% by volume or more, and an upper limit is not limited.

As for the concentration of HMF acetal introduced to the reactor in the reaction solvent, a lower limit is usually 1% by mass, preferably 5% by mass, more preferably 10% by mass, still more preferably 20% by mass, particularly preferably 30% by mass, and an upper limit is usually 60% by mass, preferably 50% by mass. By controlling the concentration of HMF acetal to be in this range, it is made unnecessary to use an excessively large reaction vessel, and the reaction is allowed to proceed in a short time with a high production efficiency. In addition, side reactions such as decomposition reaction of HMF acetal can be inhibited.

When the oxidation in this step is oxidative esterification, an ester of a formylfurancarboxylic acid (FFCA) acetal is generated by allowing an alcohol to coexist in the reaction system. A hydroxyalkyl ester of FFCA acetal is generated when the alcohol allowed to coexist is the diol added in the acetalization step, while an alkyl ester of the acetal is generated when the alcohol is a monool. The alcohol allowed to coexist is not particularly limited, and may be an aliphatic monoalcohol having 1 to 20 carbon atoms such as methanol, ethanol, 1-propanol, 2-propanol, 1-butanol, 2-butanol, 2-methyl-1-propanol, 2-methyl-2-propanol, 1-pentanol, 2-pentanol, 3-pentanol, 3-methyl-1-butanol, 2,2-dimethyl-1-propanol, 1-hexanol, 2-hexanol, 3-hexanol, 2-methyl-1-pentanol, 1-heptanol, 2-heptanol, 1-octanol, 2-octanol, 3-octanol, 2-ethyl-1-hexanol, 1-nonanol, 1-decanol, 1-undecyl alcohol, 1-lauryl alcohol, 1-tridecyl alcohol, 1-tetradecanol, 1-pentadecyl alcohol, 1-hexadecanol, *cis*-9-hexadecen-1-ol, 1-heptadecanol, 1-octadecanol, 16-methylheptadecen-1-ol, nonadecyl alcohol, or arachidyl alcohol, or an aliphatic diol having 2 to 8 carbon atoms such as ethylene glycol, 1,3-propanediol, 1,4-butanediol, 1,5-pentanediol, 1,6-hexanediol, or 1,4-cyclohexane dimethanol.

A reaction solution obtained after the oxidation of HMF acetal contains a furan derivative having an acetal group and a carbonyl group. In this reaction solution, the concentration of the furan derivative is preferably 70% by mole or higher, more preferably 90% by mole or higher, in all compounds having a furan skeleton. In other words, yet another embodiment of the present invention is a solution that contains a furan derivative having an acetal group and a carbonyl group, in which solution the concentration of the furan derivative in all compounds having a furan skeleton is 70% by mole or higher.

The furan derivative is a compound having a high thermal stability; therefore, by controlling the concentration thereof to be in the above-described range, an effect of inhibiting the decomposition of a compound having a furan skeleton in the reaction solution is exerted in the below-described deprotection step and second oxidation step.

<Deprotection Step>

The deprotection step is the step of deprotecting the furan derivative having an acetal group and a carbonyl

group, which is obtained in the above-described oxidation step, by way of a hydrolysis reaction to synthesize diformylfuran, FFCA, or an ester thereof. By incorporating this deprotection step, FDCA and an FDCA ester can be easily produced even under a mild condition. The furan derivative can be deprotected by a hydrolysis reaction under an acidic condition.

Another aspect of the present embodiment is a method of producing a furan derivative, which method includes the deprotection step of deprotecting FFCA (formylfurancarboxylic acid) acetal having an acetal-protected formyl group, or an ester thereof to synthesize a furan derivative and, in this method, the furan derivative is at least one selected from the group consisting of a formylfurancarboxylic acid and an ester thereof.

Methods of producing FDCA or a furandicarboxylic acid ester from HMF are disclosed in Patent Documents 3 and 4 and the like; however, the present inventors discovered that FDCA or an ester thereof, namely a furandicarboxylic acid ester, can be produced from HMF with a high yield by a production method in which FFCA acetal or an ester thereof is deprotected.

The deprotection step is the step of deprotecting FFCA acetal or an ester thereof by way of a hydrolysis reaction to synthesize FFCA or an ester thereof. By incorporating this step, FDCA and an FDCA ester can be easily produced even under a mild condition. The deprotection of FFCA acetal or an ester thereof can be performed by a hydrolysis reaction under an acidic condition.

In the present embodiment, FFCA or an ester thereof is synthesized by deprotecting an FFCA acetal or an ester thereof in the deprotection step. The FFCA acetal or the ester thereof that is used in the deprotection step is not particularly limited; however, it is preferably a compound that has a cyclic acetal structure having a 5-membered ring or a 6-membered ring. The FFCA acetal is preferably a compound represented by the following Formula (3):

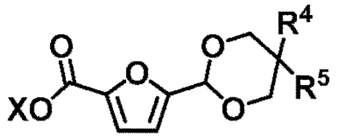 (3)

In Formula (3), X, $R^4$, and $R^5$ are independently selected from a hydrogen atom, an alkyl group having 1 to 6 carbon atoms, and an aryl group having 6 to 12 carbon atoms that optionally has an alkyl substituent. Examples of the alkyl substituent include alkyl groups having 1 to 6 carbon atoms.

The reaction in the deprotection step can be performed by, for example, a batch method, a flow method or the like. As for a liquid-contact material of a reactor, its flow channels, and the like, the material of the reactor is not particularly limited to, for example, glass, stainless steel (SUS), iron, or other metal; however, a reactor made of glass or stainless steel (SUS) is preferably used because of its corrosion resistance and high energy transfer efficiency. Further, from the standpoint of the cost of industrial production equipment, it is preferred to use a reactor made of stainless steel (SUS).

The hydrolysis conditions are not particularly limited as long as the hydrolysis of the above-described furan derivative proceeds, and the pH is preferably in a range of 0.1 to 7.

The reaction temperature in the hydrolysis is preferably room temperature to 120°C. A lower limit of the reaction temperature is more preferably 30°C, still more preferably 40°C, and an upper limit of the reaction temperature is more preferably 100°C, still more preferably 80°C. By controlling the reaction temperature to be in this range, an increase in the reaction time, concurrence of a side reaction, reaction runaway, coloring of the resulting product, and the like can be inhibited.

The reaction time in the hydrolysis is usually 1 minute to 30 hours, preferably 10 minutes to 20 hours, more preferably 30 minutes to 15 hours, taking the point when stirring is initiated as the starting point in the case of a batch method. By controlling the reaction time to be in this range, concurrence of a side reaction, coloring of the resulting product, and the like can be inhibited. It is noted here that the reaction time is not limited to this in the case of employing a flow method.

The reaction pressure in the hydrolysis is usually, but not particularly limited to, normal pressure.

As a solvent in the hydrolysis, water is usually used; however, a mixed solvent of water and an organic solvent may be used as well. From the standpoint of hydrolysis efficiency and cost advantage, it is preferred to use water as a reaction solvent.

In the case of using a mixed solvent of water and an organic solvent, the organic solvent is not particularly limited, and examples thereof include: ethers having 4 to 20 carbon atoms, such as diethyl ether, tetrahydrofuran (THF), tetrahydropyran, methyltetrahydropyran, dipropyl ether, diisopropyl ether, methyl-tert-butyl ether, butyl ether, pentyl ether, hexyl ether, octyl ether, nonyl ether, decyl ether, ethylene glycol dimethyl ether, ethylene glycol diethyl ether, diethylene glycol diethyl ether, and triethylene glycol dimethyl ether; ketones having 4 to 20 carbon atoms, such as 2-butanone (methyl ethyl ketone), 2-pentanone, 3-pentanone, 2-hexanone, 3-hexanone, cyclohexanone, 4-methyl-2-pentanone (methyl isobutyl ketone), 2-heptanone, 5-methyl-2-hexanone, 2,4-dimethyl pentanone, 5-nonanone, 4-decanone, 5-decanone, 2-undecanone, 4-undecanone, 3-dodecanone, 2-tridecanone, 2-tetradecanone, 4-tetradecanone, 2-pentadecanone, 3-pentadecanone, 7-pentadecanone, 2-hexadecanone, 3-hexadecanone, 4-hexadecanone, 6-hexadecanone, 2-heptadecanone, 4-heptadecanone, 9-heptadecanone, 3-octadecanone, and acetophenone; esters, such as ethyl acetate, propyl acetate, and butyl acetate; monoalcohols having 1 to 20 carbon atoms, such as methanol, ethanol, 1-propanol, 2-propanol, 1-butanol, 2-butanol, 2-methyl-1-propanol, 2-methyl-2-propanol, 1-pentanol, 2-pentanol, 3-pentanol, 3-methyl-1-butanol, 2,2-dimethyl-1-propanol, 1-hexanol, 2-hexanol, 3-hexanol, 2-methyl-1-pentanol, 1-heptanol, 2-heptanol, 1-octanol, 2-octanol, 3-octanol, 2-ethyl-1-hexanol, 1-nonanol, 1-decanol, 1-undecyl alcohol, 1-lauryl alcohol, 1-tridecyl alcohol, 1-tetradecanol, 1-pentadecyl alcohol, 1-hexadecanol, *cis*-9-hexadecen-1-ol, 1-heptadecanol, 1-octadecanol, 16-methylheptadecen-1-ol, nonadecyl alcohol, and arachidyl alcohol; phenols having 6 to 12 carbon atoms, such as phenol, methyl phenol, ethyl phenol, propyl phenol, and butyl phenol; saturated aliphatic hydrocarbon compounds having 3 to 12 carbon atoms, such as pentane, hexane, cyclohexane, heptane, octane, nonane, decane, dodecane, and isododecane; aromatic hydrocarbons, such as toluene, xylene, trimethylbenzene, 1,2,3,4-tetrahydronaphthalene, and 1-methylnaphthalene; lactones, such as $\gamma$-butyrolactone and $\gamma$-valerolactone; halogenated hydrocarbons, such as dichloromethane, chloroform, dichloroethane, tetrachloroethane, and hexachloroethane; and dimethylacetamide, *N,N*-dimethylformamide, *N*-methylmorpholine, *N*-methyl-2-pyrrolidinone, 1,3-dimethyl-2-imidazolidinone, hexamethylphosphoric triamide, tetramethyl urea, dimethyl sulfoxide, sulfolane, isosorbide, isosorbide dimethyl ether, propylene carbonate, and mixtures thereof. In addition, for example, an ionic liquid of a pyrrolidinium salt such as 1-butyl-1-methylpyrrolidinium bromide, a piperidinium salt such as 1-butyl-1-methylpiperidinium triflate, a pyridinium salt such as 1-butylpyridinium tetrafluoroborate, an imidazolium salt such as 1-ethyl-3-methylimidazolium chloride, an ammonium salt such as tetrabutylammonium chloride, a phosphonium salt such as tetrabutylphosphonium bromide, a sulfonium salt such as triethylsulfonium bis(trifluoromethanesulfonyl)imide, or the like may be used as well.

Examples of a preferred catalyst for the hydrolysis include: acidic cation exchange resins, such as AMBERLYST, AMBERLITE, and DIAION; metal oxides and metal composite oxides, such as zeolite, $Nb_2O_5$, $YNbO_4$, $Ta_2O_5$, $Nb_2O_5$-$WO_3$, $Nb_2O_5$-$MoO_3$, $TiO_2$-$WO_3$, $TiO_2$-$MoO_3$, $SiO_2$-$Nb_2O_5$, and $SiO_2$-$Ta_2O_5$; sulfuric acid immobilization catalysts, such as clay and sulfated $Z_1O_2$; and phosphoric acid immobilization catalysts, such as phosphoric acid-treated titania and niobia. Thereamong, from the standpoint of selectively allowing only the deprotection of acetal of HMF acetal to proceed in a water-containing solvent, acidic cation exchange resins, such as AMBERLYST, AMBERLITE, and DIAION, as well as zeolite are more preferred, and acidic cation exchange resins such as AMBERLYST, AMBERLITE, and DIAION are particularly preferred.

A reaction solution obtained by the hydrolysis of the furan derivative contains diformylfuran, FFCA, or an ester thereof. In this reaction solution, the concentration of diformylfuran, the FFCA, or the ester thereof is preferably 70% by mole or higher, more preferably 90% by mole or higher, in all compounds having a furan skeleton. Particularly, the FFCA or the ester thereof is a compound having a high thermal stability; therefore, by controlling the concentration thereof to be in the above-described range, the decomposition of a compound having a furan skeleton in the reaction solution can be inhibited in the below-described second oxidation step.

<Recovery Step>

The reaction solution, which is obtained by deprotecting the furan derivative having an acetal group and a carbonyl group and thereby synthesizing diformylfuran, FFCA, or an ester thereof in the deprotection step, may be subjected to the recovery step to recover a diol from the reaction solution. This diol to be recovered is the diol added in the above-described acetalization step. A method of recovering the diol from the reaction solution is not particularly limited, and the diol can be recovered by extraction, distillation, or the like.

In the case of performing extraction in the recovery step, the temperature thereof is preferably room temperature to 90°C. A lower limit of this temperature is more preferably 30°C, and an upper limit is more preferably 60°C, still more preferably 40°C. By controlling the reaction temperature to be in this range, the extraction efficiency at the time of the recovery is improved, so that the operating time can be shortened.

As an extraction solvent, an organic solvent is usually used; however, a mixed solvent of water and an organic solvent may be used as well. From the standpoint of cost advantage, it is preferred to use a single solvent as the extraction solvent.

The organic solvent is not particularly limited, and examples of the organic solvent that may be used include: ethers having 4 to 20 carbon atoms, such as diethyl ether, tetrahydropyran, methyltetrahydropyran, dipropyl ether, diisopropyl ether, methyl-tert-butyl ether, butyl ether, pentyl ether, hexyl ether, octyl ether, nonyl ether, decyl ether, ethylene glycol dimethyl ether, ethylene glycol diethyl ether, diethylene glycol diethyl ether, and triethylene glycol dimethyl ether; ketones having 4 to 20 carbon atoms, such as 2-butanone (methyl ethyl ketone), 2-pentanone, 3-pentanone, 2-hexanone, 3-hexanone, cyclohexanone, 4-methyl-2-pentanone (methyl isobutyl ketone), 2-heptanone, 5-methyl-2-hexanone, 2,4-dimethyl pentanone, 5-nonanone, 4-decanone, 5-decanone, 2-undecanone, 4-undecanone, 3-dodecanone, 2-tridecanone, 2-tetradecanone, 4-tetradecanone, 2-pentadecanone, 3-pentadecanone, 7-pentadecanone, 2-hexadecanone, 3-hexadecanone, 4-hexadecanone, 6-hexadecanone, 2-heptadecanone, 4-heptadecanone, 9-heptadecanone,

3-octadecanone, and acetophenone; esters, such as ethyl acetate, propyl acetate, and butyl acetate; saturated aliphatic hydrocarbon compounds having 3 to 12 carbon atoms, such as pentane, hexane, cyclohexane, heptane, octane, nonane, decane, dodecane, and isododecane; aromatic hydrocarbons, such as toluene, xylene, trimethylbenzene, 1,2,3,4-tetrahydronaphthalene, and 1-methylnaphthalene; lactones, such as $\gamma$-butyrolactone and $\gamma$-valerolactone; and halogenated hydrocarbons, such as dichloromethane, chloroform, dichloroethane, tetrachloroethane, and hexachloroethane. Thereamong, the organic solvent is preferably at least one selected from the group consisting of ethers, ketones, esters, lactones, aromatic hydrocarbons, halogenated hydrocarbons, and saturated aliphatic hydrocarbons, more preferably at least one selected from the group consisting of ethers, ketones, aromatic hydrocarbons, and saturated aliphatic hydrocarbons, still more preferably an ester. As the ester, specifically, ethyl acetate is preferred.

The operations in the recovery step can be performed by, for example, a batch method or a flow method. As for a liquid-contact material of a reactor, its flow channels, and the like, the material of the reactor is not particularly limited to, for example, glass, stainless steel (SUS), iron, or other metal; however, a reactor made of glass or stainless steel (SUS) is preferably used because of its corrosion resistance and high energy transfer efficiency. Further, from the standpoint of the cost of industrial production equipment, it is preferred to use a reactor made of stainless steel (SUS).

<Second Oxidation Step>

The second oxidation step is the step of oxidizing diformylfuran, the FFCA, or the ester thereof that is obtained in the above-described deprotection step. It is noted here that the oxidation in this step includes oxidative esterification. FDCA can be obtained by oxidation of the above-described compound, while an FDCA ester can be obtained by oxidative esterification of the above-described compound.

Yet another aspect of the present embodiment is a method of producing a furandicarboxylic acid or an ester thereof, which method includes the second oxidation step of bringing a composition containing a furan derivative into contact with an oxidation catalyst and, in this method, the furan derivative is at least one selected from the group consisting of diformylfuran, a formylfurancarboxylic acid, and an ester thereof, and the concentration of the furan derivative in all compounds having a furan skeleton that is contained in the composition containing the furan derivative is 70% by mole or higher.

Methods of producing FDCA or a furandicarboxylic acid ester from HMF are disclosed in Patent Documents 3 and 4 and the like; however, the present inventors discovered that FDCA or a furandicarboxylic acid ester can be produced from HMF with a high yield by a production method that includes the step of oxidizing the above-described furan derivative at a high concentration.

In the composition subjected to the second oxidation step, the concentration of diformylfuran, the FFCA, or the ester thereof is preferably 70% by mole or higher, more preferably 90% by mole or higher, in all compounds having a furan skeleton. Among the compounds having a furan skeleton, particularly the FFCA and the ester thereof are compounds having a high thermal stability; therefore, by controlling the concentration thereof to be in the above-described range, an effect of inhibiting the decomposition of a compound having a furan skeleton in the reaction solution is exerted.

In the second oxidation step, since oxidation of formyl group readily proceeds, from the standpoint of inhibiting the concurrence of a side reaction, the reaction can be performed at the reaction temperature of the above-described second oxidation step or lower. Specifically, the reaction temperature may be 80°C or lower, or 60°C or lower. A lower limit value of the reaction temperature in the second oxidation step may be the same as that of the reaction temperature in the above-described oxidation step. In this manner, in the second oxidation step, an oxidation reaction can be performed under a milder condition than the above-described oxidation step.

The reaction conditions in the second oxidation step other than the reaction temperature are the same as those conditions described above for the first oxidation step.

<Method of Recovering FDCA and FDCA Ester>

A method of recovering FDCA and an FDCA ester obtained in the second oxidation step is not particularly limited, and these products can be recovered by a method of removing the solvent by distillation, a method of extracting the products with an organic solvent after distillation of the solvent, a distillation method, a sublimation method, a crystallization method, or a chromatography method. Among these methods, a method of recovering the products while purifying them by a distillation method is preferred.

The present embodiment also encompasses the following production method as yet another aspect.

In other words, yet another aspect of the present embodiment is a method of producing a furandicarboxylic acid or a furandicarboxylic acid ester from hydroxymethylfurfural, which method includes the following production steps (i) to (iv):

(i) the acetalization step of acetalizing hydroxymethylfurfural to synthesize hydroxymethylfurfural acetal;

(ii) the first oxidation step of oxidizing the hydroxymethylfurfural acetal to synthesize a formylfurancarboxylic acid acetal or an ester thereof;
(iii) the deprotection step of deprotecting the formylfurancarboxylic acid acetal or ester thereof to synthesize a formylfurancarboxylic acid or an ester thereof; and
(iv) the second oxidation step of oxidizing the formylfurancarboxylic acid or ester thereof.

The method may further include the below-described recovery step after the step (iii).
The above-described formylfurancarboxylic acid acetal or ester thereof is preferably a compound represented by Formula (3):

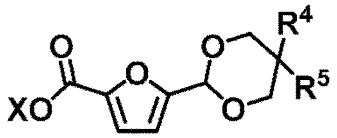
(3)

(wherein, X, $R^2$, and $R^3$ are independently selected from a hydrogen atom, an alkyl group having 1 to 6 carbon atoms, and an aryl group having 6 to 12 carbon atoms that optionally has an alkyl substituent).
Further, in the step (i), it is preferred to use an organic solvent selected from 2-butanone (methyl ethyl ketone), 4-methyl-2-pentanone (methyl isobutyl ketone), toluene, and methyltetrahydropyran, and it is preferred that the method include the recovery step of recovering a diol from a reaction solution after the step (iii), and that the diol be a diol having 2 to 10 carbon atoms.

EXAMPLES

The present invention will now be described more concretely by way of Examples; however, the present invention is not limited to the below-described Examples within the gist of the present invention.

«Example A»

With regard to the yield of a product, a solution obtained after reactions was diluted, and the product was quantitatively measured by an absolute calibration curve method based on liquid chromatography (LC-2010 manufactured by Shimadzu Corporation, column: ULTRON PS-80H). It is noted here that a differential refractive index detector (RI detector) was used as a detector, and water was used as a developing solvent. The yield of 5-hydroxymethylfurfural (5-HMF) was calculated using the following equation:

$$\text{Selectivity (\%) of 5-HMF} = \text{Number of moles of generated 5-HMF/Number of moles of glucose consumed in reaction} \times 100$$

$$\text{Yield (\%) of 5-HMF} = \text{Number of moles of generated 5-HMF/Number of moles of glucose used in reaction} \times 100$$

[Example A1]

Glucose (0.3 g, 1.7 mmol), aluminum oxide ($Al_2O_3$) (0.3 g, 100% by weight with respect to the substrate) as an isomerization catalyst, and a high-porous strongly acidic cation exchange resin (DIAION™ RCP160M) (0.6 g, 200% by weight with respect to the substrate) as a dehydration catalyst were put into a pressure-resistant vessel, methyltetrahydropyran (MTHP, 7 g) and water (3 g) were added thereto, and the vessel was tightly sealed. This vessel was heated to 120°C to heat the materials for 8 hours with stirring under a pressure of 0.2 MPa. After this 8-hour heating with stirring, cold water was introduced to the vessel to quench the reaction, and the catalysts were separated by filtration, after which an organic phase was recovered by liquid separation. Subsequently, the organic solvent was removed by distillation to obtain 5-hydroxymethylfurfural (5-HMF). The reaction conditions as well as the selectivity and the yield of 5-HMF are shown in Table 2.

[Example A2]

Glucose (0.3 g, 1.7 mmol), aluminum oxide ($Al_2O_3$) (0.3 g, 100% by weight with respect to the substrate) as an isomerization catalyst, and a high-porous strongly acidic cation exchange resin (DIAION™ RCP160M) (0.6 g, 200% by weight with respect to the substrate) as a dehydration catalyst were put into a pressure-resistant vessel, methyl isobutyl ketone (MIBK, 7 g) and water (3 g) were added thereto, and the vessel was tightly sealed. This vessel was heated to 120°C to heat the materials for 8 hours with stirring under a pressure of 0.2 MPa. After this 8-hour heating with stirring, cold water was introduced to the vessel to quench the reaction, and the catalysts were separated by filtration, after which an organic phase was recovered by liquid separation. Subsequently, the organic solvent was removed by distillation to obtain 5-hydroxymethylfurfural (5-HMF). The reaction conditions as well as the selectivity and the yield of 5-HMF are shown in Table 2.

[Example A3]

Glucose (1.0 g, 5.7 mmol), aluminum oxide ($Al_2O_3$) (1.0 g, 100% by weight with respect to the substrate) as an isomerization catalyst, and a high-porous strongly acidic cation exchange resin (DIAION™ RCP160M) (2.0 g, 200% by weight with respect to the substrate) as a dehydration catalyst were put into a pressure-resistant vessel, methyltetrahydropyran (MTHP, 7 g) and water (3 g) were added thereto, and the vessel was tightly sealed. This vessel was heated to 120°C to heat the materials for 8 hours with stirring. After this 8-hour heating with stirring, cold water was introduced to the vessel to quench the reaction, and the catalysts were separated by filtration, after which an organic phase was recovered by liquid separation. Subsequently, the organic solvent was removed by distillation to obtain 5-hydroxymethylfurfural (5-HMF). The reaction conditions as well as the selectivity and the yield of 5-HMF are shown in Table 2.

[Example A4]

Glucose (1.0 g, 5.7 mmol), aluminum oxide ($Al_2O_3$) (1.0 g, 100% by weight with respect to the substrate) as an isomerization catalyst, and a porous strongly acidic cation exchange resin (AMBERLYST (registered trademark) 15) (2.0 g, 200% by weight with respect to the substrate) as a dehydration catalyst were put into a pressure-resistant vessel, methyltetrahydropyran (MTHP, 7 g) and water (3 g) were added thereto, and the vessel was tightly sealed. This vessel was heated to 120°C to heat the materials for 8 hours with stirring. After this 8-hour heating with stirring, cold water was introduced to the vessel to quench the reaction, and the catalysts were separated by filtration, after which an organic phase was recovered by liquid separation. Subsequently, the organic solvent was removed by distillation to obtain 5-hydroxymethylfurfural (5-HMF). The reaction conditions as well as the selectivity and the yield of 5-HMF are shown in Table 2.

[Example A5]

Glucose (1.0 g, 5.7 mmol), magnesium oxide (MgO) (1.0 g, 100% by weight with respect to the substrate) as an isomerization catalyst, and a high-porous strongly acidic cation exchange resin (DIAION™ RCP160M) (2.0 g, 200% by weight with respect to the substrate) as a dehydration catalyst were put into a pressure-resistant vessel, methyltetrahydropyran (MTHP, 7 g) and water (3 g) were added thereto, and the vessel was tightly sealed. This vessel was heated to 120°C to heat the materials for 8 hours with stirring. After this 8-hour heating with stirring, cold water was introduced to the vessel to quench the reaction, and the catalysts were separated by filtration, after which an organic phase was recovered by liquid separation. Subsequently, the organic solvent was removed by distillation to obtain 5-hydroxymethylfurfural (5-HMF). The reaction conditions as well as the selectivity and the yield of 5-HMF are shown in Table 2.

[Example A6]

Glucose (1.0 g, 5.7 mmol), magnesium oxide (MgO) (1.0 g, 100% by weight with respect to the substrate) as an isomerization catalyst, and a phosphoric acid-activated carbon (1.0 g, 100% by weight with respect to the substrate) as a dehydration catalyst were put into a pressure-resistant vessel, methyltetrahydropyran (MTHP, 7 g) and water (3 g) were added thereto, and the vessel was tightly sealed. This vessel was heated to 140°C to heat the materials for 8 hours with stirring. After this 8-hour heating with stirring, cold water was introduced to the vessel to quench the reaction, and the catalysts were separated by filtration, after which an organic phase was recovered by liquid separation. Subsequently, the organic solvent was removed by distillation to obtain 5-hydroxymethylfurfural (5-HMF). The reaction conditions as well as the selectivity and the yield of 5-HMF are shown in Table 2.

[Example A7]

Glucose (1.0 g, 5.7 mmol), magnesium oxide (MgO) (1.0 g, 100% by weight with respect to the substrate) as an isomerization catalyst, and a phosphoric acid-modified silica gel (1.0 g, 100% by weight with respect to the substrate) as a dehydration catalyst were put into a pressure-resistant vessel, methyltetrahydropyran (MTHP, 7 g) and water (3 g) were added thereto, and the vessel was tightly sealed. This vessel was heated to 140°C to heat the materials for 8 hours with stirring. After this 8-hour heating with stirring, cold water was introduced to the vessel to quench the reaction, and the catalysts were separated by filtration, after which an organic phase was recovered by liquid separation. Subsequently, the organic solvent was removed by distillation to obtain 5-hydroxymethylfurfural (5-HMF). The reaction conditions as well as the selectivity and the yield of 5-HMF are shown in Table 2.

[Comparative Example A1]

Glucose (0.3 g, 1.7 mmol) and phosphoric acid (15 mg, 5% by weight with respect to the substrate) as a catalyst were put into a pressure-resistant vessel, methyl isobutyl ketone (MIBK, 7 g) and water (3 g) were added thereto, and the vessel was tightly sealed. This vessel was heated to 140°C to heat the materials for 4 hours with stirring. After this 4-hour heating with stirring, cold water was introduced to the vessel to quench the reaction, and the catalyst was separated by filtration, after which an organic phase was recovered by liquid separation. Subsequently, the organic solvent was removed by distillation to obtain 5-hydroxymethylfurfural (5-HMF). The reaction conditions as well as the selectivity and the yield of 5-HMF are shown in Table 2.

[Comparative Example A2]

Glucose (0.3 g, 1.7 mmol) and phosphoric acid (15 mg, 5% by weight with respect to the substrate) as a catalyst were put into a pressure-resistant vessel, methyltetrahydropyran (MTHP, 7 g) and water (3 g) were added thereto, and the vessel was tightly sealed. This vessel was heated to 140°C to heat the materials for 4 hours with stirring. After this 4-hour heating with stirring, cold water was introduced to the vessel to quench the reaction, and the catalyst was separated by filtration, after which an organic phase was recovered by liquid separation. Subsequently, the organic solvent was removed by distillation to obtain 5-hydroxymethylfurfural (5-HMF). The reaction conditions as well as the selectivity and the yield of 5-HMF are shown in Table 2.

[Comparative Example A3]

Glucose (1.0 g, 5.7 mmol), a high-porous strongly acidic cation exchange resin (DIAION™ RCP160M) (2.0 g, 200% by weight with respect to the substrate) as a dehydration catalyst, and sodium chloride (NaCl) (1.0 g, 100% by weight with respect to the substrate) as an additive were put into a pressure-resistant vessel, methyl isobutyl ketone (MIBK, 7 g) and water (3 g) were added thereto, and the vessel was tightly sealed. This vessel was heated to 120°C to heat the materials for 8 hours with stirring. After this 8-hour heating with stirring, cold water was introduced to the vessel to quench the reaction, and the catalyst was separated by filtration, after which an organic phase was recovered by liquid separation. Subsequently, the organic solvent was removed by distillation to obtain 5-hydroxymethylfurfural (5-HMF). The reaction conditions as well as the selectivity and the yield of 5-HMF are shown in Table 2.

[Comparative Example A4]

Glucose (1.0 g, 5.7 mmol), aluminum oxide ($Al_2O_3$) (1.0 g, 100% by weight with respect to the substrate) as an isomerization catalyst, a high-porous strongly acidic cation exchange resin (DIAION™ RCP160M) (2.0 g, 200% by weight with respect to the substrate) as a dehydration catalyst, and sodium chloride (NaCl) (1.0 g, 100% by weight with respect to the substrate) as an additive were put into a pressure-resistant vessel, dimethyl sulfoxide (DMSO, 10 g) was added thereto, and the vessel was tightly sealed. This vessel was heated to 120°C to heat the materials for 8 hours with stirring. After this 8-hour heating with stirring, cold water was introduced to the vessel to quench the reaction, and the catalysts were separated by filtration, after which an organic phase was recovered. Subsequently, the organic solvent was removed by distillation to obtain 5-hydroxymethylfurfural (5-HMF). The reaction conditions as well as the selectivity and the yield of 5-HMF are shown in Table 2.

[Comparative Example A5]

Glucose (1.0 g, 5.7 mmol), aluminum oxide ($Al_2O_3$) (1.0 g, 100% by weight with respect to the substrate) as an isomerization catalyst, and a high-porous strongly acidic cation exchange resin (DIAION™ RCP160M) (2.0 g, 200%

by weight with respect to the substrate) as a dehydration catalyst were put into a pressure-resistant vessel, dimethyl sulfoxide (DMSO, 10 g) was added thereto, and the vessel was tightly sealed. This vessel was heated to 120°C to heat the materials for 8 hours with stirring. After this 8-hour heating with stirring, cold water was introduced to the vessel to quench the reaction, and the catalysts were separated by filtration, after which an organic phase was recovered. Subsequently, the organic solvent was removed by distillation to obtain 5-hydroxymethylfurfural (5-HMF). The reaction conditions as well as the selectivity and the yield of 5-HMF are shown in Table 2.

[Comparative Example A6]

Glucose (1.0 g, 5.7 mmol), aluminum oxide ($Al_2O_3$) (1.0 g, 100% by weight with respect to the substrate) as an isomerization catalyst, and a porous strongly acidic cation exchange resin (AMBERLYST (registered trademark) 15) (2.0 g, 200% by weight with respect to the substrate) as a dehydration catalyst were put into a pressure-resistant vessel, dimethyl sulfoxide (DMSO, 10 g) was added thereto, and the vessel was tightly sealed. This vessel was heated to 120°C to heat the materials for 8 hours with stirring. After this 8-hour heating with stirring, cold water was introduced to the vessel to quench the reaction, and the catalysts were separated by filtration, after which an organic phase was recovered. Subsequently, the organic solvent was removed by distillation to obtain 5-hydroxymethylfurfural (5-HMF). The reaction conditions as well as the selectivity and the yield of 5-HMF are shown in Table 2.

[Comparative Example A7]

Glucose (0.3 g, 1.7 mmol), aluminum oxide ($Al_2O_3$) (1.0 g, 100% by weight with respect to the substrate) as an isomerization catalyst, a high-porous strongly acidic cation exchange resin (DIAION™ RCP160M) (2.0 g, 200% by weight with respect to the substrate) as a dehydration catalyst, and sodium chloride (NaCl) (4.0 g, 400% by weight with respect to the substrate) as an additive were put into a pressure-resistant vessel, methyltetrahydropyran (MTHP, 7 g) and water (3 g) were added thereto, and the vessel was tightly sealed. This vessel was heated to 120°C to heat the materials for 8 hours with stirring. After this 8-hour heating with stirring, cold water was introduced to the vessel to quench the reaction, and the catalysts were separated by filtration, after which an organic phase was recovered by liquid separation. Subsequently, the organic solvent was removed by distillation to obtain 5-hydroxymethylfurfural (5-HMF). The reaction conditions as well as the selectivity and the yield of 5-HMF are shown in Table 2.

[Comparative Example A8]

Glucose (0.1 g, 0.5 mmol), aluminum oxide ($Al_2O_3$) (2.0 g, 200% by weight with respect to the substrate) as an isomerization catalyst, a high-porous strongly acidic cation exchange resin (DIAION™ RCP160M) (2.0 g, 200% by weight with respect to the substrate) as a dehydration catalyst, and sodium chloride (NaCl) (4.0 g, 400% by weight with respect to the substrate) as an additive were put into a pressure-resistant vessel, methyl isobutyl ketone (MIBK, 7 g), *N*-methylpyrrolidone (NMP, 2.4 g), and water (0.6 g) were added thereto, and the vessel was tightly sealed. This vessel was heated to 120°C to heat the materials for 8 hours with stirring. After this 8-hour heating with stirring, cold water was introduced to the vessel to quench the reaction, and the catalysts were separated by filtration, after which an organic phase was recovered by liquid separation. Subsequently, the organic solvent was removed by distillation to obtain 5-hydroxymethylfurfural (5-HMF). The reaction conditions as well as the selectivity and the yield of 5-HMF are shown in Table 2.

[Comparative Example A9]

Glucose (0.1 g, 0.5 mmol), aluminum oxide ($Al_2O_3$) (2.0 g, 200% by weight with respect to the substrate) as an isomerization catalyst, a high-porous strongly acidic cation exchange resin (DIAION™ RCP160M) (2.0 g, 200% by weight with respect to the substrate) as a dehydration catalyst, and sodium chloride (NaCl) (1.0 g, 100% by weight with respect to the substrate) as an additive were put into a pressure-resistant vessel, methyl isobutyl ketone (MIBK, 7 g), *N*-methylpyrrolidone (NMP, 2.4 g), and water (0.6 g) were added thereto, and the vessel was tightly sealed. This vessel was heated to 120°C to heat the materials for 8 hours with stirring. After this 8-hour heating with stirring, cold water was introduced to the vessel to quench the reaction, and the catalysts were separated by filtration, after which an organic phase was recovered by liquid separation. Subsequently, the organic solvent was removed by distillation to obtain 5-hydroxymethylfurfural (5-HMF). The reaction conditions as well as the selectivity and the yield of 5-HMF are shown in Table 2.

[Table 2]

| | Glucose (mmol) | Water (g) | Organic solvent (g) | Isomerization catalyst | | Dehydration catalyst | | Additive (wt%) | Tempera ture (°C) | Time (h) | Hydroxymethylfurfural | |
|---|---|---|---|---|---|---|---|---|---|---|---|---|
| | | | | Amount (wt%) | Speoifio surface area ($m^2/g$) | Amount (wt%) | Specific surface area ($m^2/g$) | | | | Selectivity (%) | Yield (%) |
| Example 1 | 1.7 | 3.0 | Methyltetrahydropyran 7.0 | $Al_2O_3$ 100 | 100 | RCP160M 200 | 49 | - | 120 | 8 | 95 | 80 |
| Example 2 | 1.7 | 3.0 | Methyl isobutyl ketone 7.0 | $Al_2O_3$ 100 | 100 | RCP160M 200 | 49 | - | 120 | 8 | 94 | 77 |
| Example 3 | 5.7 | 3.0 | Methyltetrahydropyran 7.0 | $Al_2O_3$ 100 | 100 | RCP160M 200 | 49 | - | 120 | 8 | 96 | 71 |
| Example 4 | 5.7 | 3.0 | Methyltetrahydropyran 7.0 | $Al_2O_3$ 100 | 70 | Amberlyst15 200 | 35 | - | 120 | 8 | 95 | 69 |
| Example 5 | 5.7 | 3.0 | Methyltetrahydropyran 7.0 | MgO 100 | 86 | RCP160M 200 | 49 | - | 120 | 8 | 94 | 86 |
| Example 6 | 5.7 | 3.0 | Methyltetrahydropyran 7.0 | MgO 100 | 85 | Phosphoric acid-activated carbon 100 | 400 | - | 140 | 8 | 97 | 51 |
| Example 7 | 5.7 | 3.0 | Methyltetrahydropyran 7.0 | MgO 100 | 86 | Phosphoric acid-modified oarbon 100 | 450 | - | 140 | 8 | 96 | 66 |
| Comparative Example 1 | 1.7 | 3.0 | Methyl isobutyl ketone 7.0 | - | - | Phosphoric acid 6 | - | - | 140 | 4 | 53 | 31 |
| Comparative Example 2 | 1.7 | 3.0 | Methyltetrahydropyran 7.0 | - | - | Phosphoric acid 5 | - | - | 140 | 4 | 61 | 28 |
| Comparative Example 3 | 5.7 | 3.0 | Methyl isobutyl ketone 7.0 | - | - | RCP160M 200 | 49 | NaCl 100 | 120 | 8 | 75 | 28 |
| Comparative Example 4 | 5.7 | - | Dimethyl sulfoxid 10.0 | $Al_2O_3$ 100 | 70 | RCP160M 200 | 49 | NaCl 100 | 120 | 8 | 80 | 36 |
| Comparative Example 5 | 5.7 | - | Dimethyl sulfoxid 10.0 | $Al_2O_3$ 100 | 100 | RCP160M 200 | 49 | - | 120 | 8 | 72 | 37 |

(continued)

| | Glucose (mmol) | Water (g) | Organic solvent (g) | Isomerization catalyst | | Dehydration catalyst | | Additive (wt%) | Tempera ture (°C) | Time (h) | Hydroxymethylfurfural | |
|---|---|---|---|---|---|---|---|---|---|---|---|---|
| | | | | Amount (wt%) | Speoifio surface area ($m^2/g$) | Amount (wt%) | Specific surface area ($m^2/g$) | | | | Selectivity (%) | Yield (%) |
| Comparative Example 8 | 6.7 | - | Dimethyl sulfoxid 10.0 | $Al_2O_3$ 100 | 70 | Amberlyst15 200 | 36 | - | 120 | 8 | 69 | 19 |
| Comparative Example 7 | 1.7 | 3.0 | Methyltetrahydropyran 7.0 | $Al_2O_3$ 100 | 100 | RCP160M 200 | 49 | NaCl 400 | 120 | a | 55 | 49 |
| Comparative Example 8 | 0.5 | 0.6 | Methyl isobutyl ketone 7.0 N-methylpyrrolidone 2.4 | $Al_2O_3$ 200 | 70 | RCP160M 200 | 49 | NaCl 400 | 120 | 8 | 25 | 23 |
| Comparative Example 9 | 0.5 | 0.6 | Methyl isobutyl ketone 7.0 N-methylpyrrolidone 2.4 | $Al_2O_3$ 200 | 70 | RCP160M 200 | 49 | NaCl 100 | 120 | 8 | 49 | 45 |

*The catalyst amounts and the additive amounts in this table each represent a mass ratio with respect to glucose (substrate).

As shown in Table 2, in Examples A1 to A7 where both a biphasic solvent and a mixed catalyst obtained by mixing an isomerization catalyst and a dehydration catalyst were used in the process of producing 5-MHF from glucose, 5-HMF was obtained with a high yield and a high selectivity. On the other hand, in Comparative Examples A1 to A9 where a conventional method of using only a biphasic solvent or a mixed catalyst was employed, it was found that the yield and the selectivity of 5-HMF, particularly the latter, was lower than those in Examples. In addition, in Comparative Examples A7 to A9 where an addition of NaCl in an amount of 100 to 400% by weight with respect to the substrate was examined as in prior art documents, the yield and the selectivity of 5-HMF were found to be low. As asserted by the present technology, an addition of a Na salt facilitates the distribution of a product in an oil phase; however, for example, it has a large adverse effect on an isomerization catalyst and a dehydration catalyst, especially on a dehydration catalyst (reduction in acid strength).

«Example B»

With regard to the yield of a product, as an internal standard, trioxane was added to a solution obtained after reactions, and the product was quantitatively measured using a nuclear magnetic resonance (NMR) spectrometer (JOEL JNM-ECX 400, manufactured by JOEL Ltd.). It is noted here that the measurement frequency was 400 MHz, and the measurement solvent was deuterated dimethyl sulfoxide. The yield of FDCA was calculated using the following equations:

Yield (%) of HMF acetal in acetalization step = Number of moles of HMF acetal generated in acetalization step/Number of moles of HMF used in reaction in acetalization step × 100

Yield (%) of FFCA acetal in oxidation step = Number of moles of FFCA acetal generated in oxidation step/Number of moles of HMF acetal used in reaction in oxidation step × 100

Yield (%) of FFCA in deprotection step = Number of moles of FFCA generated in deprotection step/Number of moles of FFCA acetal used in reaction in deprotection step × 100

Yield (%) of FDCA in second oxidation step = Number of moles of FDCA generated in second oxidation step/Number of moles of FFCA used in reaction in second oxidation step × 100

Overall yield of FDCA (%) = (Yield (%) of HMF acetal in acetalization step/100) × (Yield (%) of FFCA acetal in oxidation step/100) × (Yield (%) of FFCA in deprotection step/100) × (Yield (%) of FDCA in second oxidation step/100) × 100

[Example B1]

<Acetalization Step>

HMF (10.0 g, 79.3 mmol), neopentyl glycol (12.4 g, 119.0 mmol) as a diol, and a porous strongly acidic cation exchange resin (AMBERLYST 15) (1.0 g, 10% by weight with respect to the substrate) as a catalyst were added to

toluene (40.0 g) used as a solvent, and the resultant was stirred at 30°C for 1 hour. Subsequently, the resulting reaction solution was cooled to room temperature, and the catalyst was separated by filtration. The resulting filtrate was washed with water, and toluene was removed by distillation, whereby HMF acetal was obtained with a yield of 97%. The reaction conditions of the acetalization step and the yield of HMF acetal are shown in Table 3.

<First Oxidation Step>

The thus obtained HMF acetal (4.0 g, 18.8 mmol), potassium carbonate (5.2 g, 37.6 mmol) as a base, and 5% palladium-supported carbon (Pd/C) (4.0 g, 100% by weight with respect to the substrate) as a catalyst were added to water (16.0 g) used as a solvent. Air was introduced thereto such that the pressure was 0.9 MPa, and the resultant was stirred at 80°C for 8 hours. Subsequently, the resulting reaction solution was cooled to room temperature, and the pressure of the reaction vessel was reduced. Thereafter, the catalyst was separated by filtration to obtain an aqueous solution containing FFCA acetal represented by the following Formula (4). The yield was 97%. It is noted here that decomposition of neopentyl glycol used as a diol was not confirmed in this step. The reaction conditions of this oxidation step and the yield of FFCA acetal are shown in Table 4.

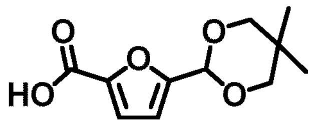

(4)

<Deprotection Step>

FFCA acetal (4.0 g, 17.7 mmol) obtained in the above-described Example and a high-porous strongly acidic cation exchange resin (DIAION™ RCP160M) (0.4 g, 10% by weight with respect to the substrate) as a catalyst were added to water (16.0 g) used as a solvent, and the resultant was stirred at 60°C for 1 hour. Subsequently, the resulting reaction solution was cooled to room temperature, and the catalyst was separated by filtration. Thereafter, the resulting filtrate was washed with ethyl acetate to obtain FFCA with a yield of 96%. It is noted here that decomposition of neopentyl glycol used as a diol was not confirmed in this step. The reaction conditions of the deprotection step and the yield of FFCA are shown in Table 5.

<Second Oxidation Step>

The thus obtained FFCA (2.4 g, 17.0 mmol), potassium carbonate (4.7 g, 34.0 mmol) as a base, and 5% palladium-supported carbon (Pd/C) (2.4 g, 100% by weight with respect to the substrate) as a catalyst were added to water (9.6 g) used as a solvent. Air was introduced thereto such that the pressure was 0.9 MPa, and the resultant was stirred at 80°C for 8 hours. Subsequently, the resulting reaction solution was cooled to room temperature, and the pressure of the reaction vessel was reduced. Thereafter, the catalyst was separated by filtration, and sulfuric acid was poured into the thus obtained filtrate, after which the resulting precipitate was recovered by filtration. The thus obtained solid was washed with water to obtain FDCA with a yield of 96%. It is noted here that the overall yield of FDCA was 87%. The reaction conditions of the second oxidation step and the yield of FDCA are shown in Table 6.

[Example B2]

<Acetalization Step>

HMF acetal was obtained with a yield of 97% in the same manner as in Example B1, except that the amount of HMF (40.0 g, 317.2 mmol), neopentyl glycol (49.6 g, 475.8 mmol), a porous strongly acidic cation exchange resin (AMBERLYST 15) (4.0 g, 10% by weight with respect to the substrate), and toluene (60.0 g) were changed. The reaction conditions of the acetalization step and the yield of HMF acetal are shown in Table 3.

<First Oxidation Step>

FFCA acetal was obtained with a yield of 97% in the same manner as in Example B1, except that the amount of HMF acetal (8.0 g, 37.7 mmol), potassium carbonate (10.4 g, 75.4 mmol), 5% palladium-supported carbon (Pd/C) (0.8 g, 10% by weight with respect to the substrate), and water (12.0 g) were changed. It is noted here that decomposition of neopentyl glycol was not confirmed in this step. The reaction conditions of this oxidation step and the yield of FFCA acetal are shown in Table 4.

<Deprotection Step>

FFCA was obtained with a yield of 95% in the same manner as in Example B1, except that the amount of water was changed to 6.0 g. It is noted here that decomposition of neopentyl glycol was not confirmed in this step. The reaction conditions of the deprotection step and the yield of FFCA are shown in Table 53.

<Second Oxidation Step>

FDCA was obtained with a yield of 97% in the same manner as in Example B1, except that the amount of FFCA (1.2 g, 8.6 mmol), potassium carbonate (2.4 g, 17.2 mmol), 5% palladium-supported carbon (Pd/C) (0.12 g, 10% by weight with respect to the substrate), and water (1.8 g) were changed. It is noted here that the overall yield of FDCA was 87%. The reaction conditions of the second oxidation step and the yield of FDCA are shown in Table 6.

[Example B3]

<Acetalization Step>

HMF acetal was obtained with a yield of 96% in the same manner as in Example B2, except that the amount of neopentyl glycol was changed to 39.6 g, 380.6 mmol. The reaction conditions of the acetalization step and the yield of HMF acetal are shown in Table 3.

<First Oxidation Step>

FFCA acetal was obtained with a yield of 97% in the same manner as in Example B2, except that 5% platinum-supported carbon (Pt/C) (0.8 g, 10% by weight with respect to the substrate) was used as a catalyst. It is noted here that decomposition of neopentyl glycol was not confirmed in this step. The reaction conditions of this oxidation step and the yield of FFCA acetal are shown in Table 4.

<Deprotection Step>

FFCA was obtained with a yield of 96% in the same manner as in Example B2, except that the reaction time was changed to 2 hours. It is noted here that decomposition of neopentyl glycol was not confirmed in this step. The reaction conditions of the deprotection step and the yield of FFCA are shown in Table 5.

<Second Oxidation Step>

FDCA was obtained with a yield of 98% in the same manner as in Reference Example 2, except that 5% platinum-supported carbon (Pt/C) (0.1 g, 10% by weight with respect to the substrate) was used as a catalyst. It is noted here that the overall yield of FDCA was 88%. The reaction conditions of the second oxidation step and the yield of FDCA are shown in Table 6.

[Comparative Reference Example B1]

HMF (0.3 g, 2.4 mmol) and 5% platinum-supported carbon (Pt/C) (0.03 g, 10% by weight with respect to the substrate) as a catalyst were added to water (2.7 g) used as a solvent. Air was introduced thereto such that the pressure was 0.9 MPa, and the resultant was stirred at 80°C for 16 hours. Subsequently, the resulting reaction solution was cooled to room temperature, and the pressure of the reaction vessel was reduced. Thereafter, the catalyst was separated by filtration, and sulfuric acid was poured into the thus obtained filtrate, after which the resulting precipitate was recovered by filtration. The thus obtained solid was washed with water to obtain FDCA with a yield of 22%. The reaction conditions and the yield of FDCA are shown in Table 7.

[Comparative Reference Example B2]

FDCA was obtained with a yield of 45% in the same manner as in Comparative Reference Example B1, except that HMF (1.2 g, 9.6 mmol), 5% platinum-supported carbon (Pt/C) (0.12 g, 10% by weight with respect to the substrate) as a catalyst, and potassium carbonate (2.65 g, 19.2 mmol) were added to water (1.8 g) used as a solvent. The reaction conditions and the yield of FDCA are shown in Table 7.

[Table 3]

| | HMF | | Substrate concentration (%) | Solvent (g) | Diol | | Catalyst | | Temperature (°C) | Time (h) | Yield of HMF acetal in acetalization step (%) |
|---|---|---|---|---|---|---|---|---|---|---|---|
| | Amount (g) | Number of moles (mmol) | | | Name | Amount (mmol) | Name | Amount with respect to substrate (wt%) | | | |
| Example B1 | 10.0 | 79.3 | 20 | Toluene 40.0 | Neopentyl glycol | 119.0 | AMBERLYST 15 | 10 | 30 | 1 | 97 |
| Example B2 | 40.0 | 317.2 | 40 | Toluene 60.0 | Neopentyl glycol | 475.8 | AMBERLYST 15 | 10 | 30 | 1 | 97 |
| Example B3 | 40.0 | 317.2 | 40 | Toluene 60.0 | Neopentyl glycol | 380.6 | AMBERLYST 15 | 10 | 30 | 1 | 98 |

[Table 4]

| | HMF acetal | | Substrate concentration (%) | Solvent (g) | Base | | Catalyst | | Introduced air (MPa) | Temperature (°C) | Time (h) | Yield of FFCA acetal in oxidation step (%) |
|---|---|---|---|---|---|---|---|---|---|---|---|---|
| | Amount (g) | Number of moles (mmol) | | | Name | Amount (mmol) | Name | Amount with respect to substrate (wt%) | | | | |
| Example B1 | 4.0 | 18.8 | 20 | Water 16.0 | Potassium carbonate | 37.6 | Pd/C | 100 | 0.9 | 80 | 8 | 97 |
| Example B2 | 8.0 | 37.7 | 40 | Water 12.0 | Potassium carbonate | 75.4 | Pd/C | 10 | 0.9 | 80 | 8 | 97 |
| Example B3 | 8.0 | 37.7 | 40 | Water 12.0 | Potassium carbonate | 75.4 | Pt/C | 10 | 0.9 | 80 | 8 | 97 |

[Table 5]

| | FFCA acetal | | Substrate concentration (%) | Solvent (g) | Catalyst | | Temperature (°C) | Time (h) | Yield of FFCA in deprotection step (%) |
|---|---|---|---|---|---|---|---|---|---|
| | Amount (g) | Number of moles (mmol) | | | Name | Amount with respect to substrate (wt%) | | | |
| Example B1 | 4.0 | 17.7 | 20 | Water 16.0 | DIAION RCP160M | 10 | 60 | 1 | 98 |
| Example B2 | 4.0 | 17.7 | 40 | Water 6.0 | DIAION RCP160M | 10 | 60 | 1 | 95 |
| Example B3 | 4.0 | 17.7 | 40 | Water 6.0 | DIAION RCP160M | 10 | 60 | 2 | 96 |

[Table 6]

| | FFCA | | Substrate concentration (%) | Solvent (g) | Base | | Catalyst | | Introduced air (MPa) | Temperature (°C) | Time (h) | Yield of FDCA in second oxidation step (%) | Overall yield of FDCA (%) |
|---|---|---|---|---|---|---|---|---|---|---|---|---|---|
| | Amount(g) | Number of moles (mmol) | | | Name | Amount (mmol) | Name | Amount with respect to substrate (wt%) | | | | | |
| Example B1 | 2.4 | 17.0 | 20 | Water 9.6 | Potassium carbonate | 34.0 | Pd/C | 100 | 0.9 | 80 | 8 | 96 | 87 |
| Example B2 | 1.2 | 8.6 | 40 | Water 1.8 | Potassium carbonate | 17.2 | Pd/C | 10 | 0.9 | 80 | 8 | 97 | 87 |
| Example B3 | 1.2 | 8.6 | 40 | Water 1.8 | Potassium carbonate | 17.2 | Pt/C | 10 | 0.9 | 80 | 8 | 98 | 88 |

[Table 7]

| | HMF | | Substrate concentration (%) | Solvent (g) | Base | | Catalyst | | Introduced air (MPa) | Temperature (°C) | Time (h) | Yield of FDCA |
|---|---|---|---|---|---|---|---|---|---|---|---|---|
| | Amount (g) | Number of moles (mmol) | | | Name | Amount (mmol) | Name | Amount with respect to substrate (wt%) | | | | |
| Comparative Reference Example 1 | 0.3 | 2.4 | 10 | Water 2.7 | - | - | Pt/C | 10 | 0.9 | 80 | 16 | 22 |
| Comparative Reference Example 2 | 1.2 | 9. 6 | 40 | Water 1.8 | Potassium carbonate | 19.2 | Pt/C | 10 | 0.9 | 80 | 16 | 45 |

As shown in Table 3, in Examples B1 to B3 where the production of FFCA acetal included the oxidation step of bringing HMF acetal into contact with an oxidation catalyst, FFCA acetal was obtained with a high yield. In addition, as shown in Table 4, in Examples B1 to B3 where the production of FFCA included the deprotection step of deprotecting FFCA acetal, FFCA was obtained with a high yield. Further, as shown in Tables 5 and 6, in Examples B1 to B3 where the thus obtained FFCA acetal was used, FDCA was obtained with a high yield. Moreover, as shown in Table 6, in Examples B1 to B3 where the production of FDCA included the second oxidation step of bringing FFCA into contact with an oxidation catalyst, FDCA was obtained with a high yield.

On the other hand, as shown in Table 7, in Comparative Reference Examples B1 and B2 where a conventional method of producing FDCA by direction oxidation of HMF was employed, it was found that the yield of FDCA was lower than in Examples B1 to B3.

It is noted here that, in the second oxidation step, an FDCA ester can be obtained by oxidative esterification of FFCA obtained in the deprotection step of these Examples.

«Example C»

[Example C1]

<Step (i): Acetalization Step>

HMF (10.0 g, 79.3 mmol), neopentyl glycol (12.4 g, 119.0 mmol) as a diol, and a porous strongly acidic cation exchange resin (AMBERLYST 15) (1.0 g, 10% by weight with respect to the substrate) as a catalyst were added to toluene (40.0 g) used as a solvent, and the resultant was stirred at 30°C for 1 hour. Subsequently, the resulting reaction solution was cooled to room temperature, and the catalyst was separated by filtration. The resulting filtrate was washed with water, and toluene was removed by distillation, whereby HMF acetal was obtained with a yield of 97%. The reaction conditions of the step (i) are shown in Table 8.

<Step (ii): First Oxidation Step>

The thus obtained HMF acetal (4.0 g, 18.8 mmol), potassium carbonate (5.2 g, 37.6 mmol) as a base, and 5% palladium-supported carbon (Pd/C) (4.0 g, 100% by weight with respect to the substrate) as a catalyst were added to water (16.0 g) used as a solvent. Air was introduced thereto at 0.9 MPa, and the resultant was stirred at 80°C for 8 hours. Subsequently, the resulting reaction solution was cooled to room temperature, and the pressure of the reaction vessel was reduced. Thereafter, the catalyst was separated by filtration to obtain FFCA acetal with a yield of 97%. It is noted here that decomposition of neopentyl glycol used as a diol was not confirmed in this step. The reaction conditions of the step (ii) are shown in Table 9.

<Step (iii): Deprotection Step>

The thus obtained FFCA acetal (4.0 g, 17.7 mmol) and a high-porous strongly acidic cation exchange resin (DIAION™ RCP160M) (0.4 g, 10% by weight with respect to the substrate) as a catalyst were added to water (16.0 g) used as a solvent, and the resultant was stirred at 60°C for 1 hour. Subsequently, the resulting reaction solution was cooled to room temperature, and the catalyst was separated by filtration. Thereafter, the resulting filtrate was washed with ethyl acetate to obtain FFCA with a yield of 96%. It is noted here that decomposition of neopentyl glycol used as a diol was not confirmed in this step. The reaction conditions of the step (iii) are shown in Table 10.

<Step (iv): Second Oxidation Step>

The thus obtained FFCA (2.4 g, 17.0 mmol), potassium carbonate (4.7 g, 34.0 mmol) as a base, and 5% palladium-supported carbon (Pd/C) (2.4 g, 100% by weight with respect to the substrate) as a catalyst were added to water (9.6 g) used as a solvent. Air was introduced thereto at 0.9 MPa, and the resultant was stirred at 80°C for 8 hours. Subsequently, the resulting reaction solution was cooled to room temperature, and the pressure of the reaction vessel was reduced. Thereafter, the catalyst was separated by filtration, and sulfuric acid was poured into the thus obtained filtrate, after which the resulting precipitate was recovered by filtration. The thus obtained solid was washed with water to obtain FDCA with a yield of 96%. It is noted here that the overall yield of FDCA was 87%. The reaction conditions of the step (iv) and the yield of FDCA are shown in Table 11.

[Example C2]

<Step (i): Acetalization Step>

HMF acetal was obtained with a yield of 97% in the same manner as in Example 1, except that the amount of neopentyl glycol was changed to 9.9 g, 95.2 mmol. The reaction conditions are shown in Table 8.

<Step (ii): First Oxidation Step>

FFCA acetal was obtained with a yield of 98% in the same manner as in Example 1, except that 5% platinum-supported carbon (Pt/C) (4.0 g, 100% by weight with respect to the substrate) was used as a catalyst. It is noted here that decomposition of neopentyl glycol used as a diol was not confirmed in this step. The reaction conditions of the step (ii) are shown in Table 9.

<Step (iii): Deprotection Step>

FFCA was obtained with a yield of 96% in the same manner as in Example 1. It is noted here that decomposition of neopentyl glycol used as a diol was not confirmed in this step. The reaction conditions of the step (iii) are shown in Table 10.

<Step (iv): Second Oxidation Step>

FDCA was obtained with a yield of 97% in the same manner as in Example 1, except that 5% platinum-supported carbon (Pt/C) (2.4 g, 100% by weight with respect to the substrate) was used as a catalyst. It is noted here that the overall yield of FDCA was 89%. The reaction conditions of the step (iv) and the yield of FDCA are shown in Table 11.

[Example C3]

<Step (i): Acetalization Step>

HMF (40.0 g, 317.2 mmol) was added to propanediol (60.0 g, 788.5 mmol) used as a solvent, and the resultant was stirred at 80°C for 12 hours. Propanediol also acts as a diol for acetalization. In this process, a zeolite membrane ZX2 (CHA-type, $SiO_2/Al_2O_3$ = 6 to 8, degree of vacuum = 12 Pa) was immersed in the resulting reaction solution, and the reaction solution was stirred while being dehydrated. Subsequently, the reaction solution was cooled to room temperature, and propanediol was removed by distillation, whereby HMF acetal was obtained with a yield of 96%. The reaction conditions of the step (i) are shown in Table 8.

<Step (ii): First Oxidation Step>

The thus obtained HMF acetal (8.0 g, 37.7 mmol), potassium carbonate (10.4 g, 75.4 mmol) as a base, and 2% gold-supported hydroxyapatite (Au/HAP) (0.8 g, 10% by weight with respect to the substrate) as a catalyst were added to water (12.0 g) used as a solvent. Air was introduced thereto at 0.9 MPa, and the resultant was stirred at 80°C for 8 hours. Subsequently, the resulting reaction solution was cooled to room temperature, and the pressure of the reaction vessel was reduced. Thereafter, the catalyst was separated by filtration to obtain FFCA acetal with a yield of 95%. It is noted here that decomposition of propanediol was not confirmed in this step. The reaction conditions of the step (ii) are shown in Table 9.

<Step (iii): Deprotection Step>

FFCA was obtained with a yield of 96% in the same manner as in Example 1, except that the amount of water was changed to 6.0 g and the reaction time was changed to 2 hours. It is noted here that decomposition of propanediol was not confirmed in this step. The reaction conditions of the step (iii) are shown in Table 10.

<Step (iv): Second Oxidation Step>

The thus obtained FFCA (1.2 g, 8.6 mmol), potassium carbonate (2.4 g, 17.2 mmol) as a base, and 2% gold-supported hydroxyapatite (Au/HAP) (0.12 g, 10% by weight with respect to the substrate) as a catalyst were added to water (1.8 g) used as a solvent. Air was introduced thereto at 0.9 MPa, and the resultant was stirred at 80°C for 8 hours.

Subsequently, the resulting reaction solution was cooled to room temperature, and the pressure of the reaction vessel was reduced. Thereafter, the catalyst was separated by filtration, and sulfuric acid was poured into the thus obtained filtrate, after which the resulting precipitate was recovered by filtration. The thus obtained solid was washed with water to obtain FDCA with a yield of 91%. It is noted here that the overall yield of FDCA was 80%. The reaction conditions of the step (iv) and the yield of FDCA are shown in Table 11.

[Example C4]

<Step (i): Acetalization Step>

HMF (40.0 g, 317.2 mmol), neopentyl glycol (49.6 g, 475.8 mmol) as a diol, and a porous strongly acidic cation exchange resin (AMBERLYST 15) (4.0 g, 10% by weight with respect to the substrate) as a catalyst were added to toluene (60.0 g) used as a solvent, and the resultant was stirred at 30°C for 1 hour. Subsequently, the resulting reaction solution was cooled to room temperature, and the catalyst was separated by filtration. The resulting filtrate was washed with water, and toluene was removed by distillation, whereby HMF acetal was obtained with a yield of 97%. The reaction conditions of the step (i) are shown in Table 8.

<Step (ii): First Oxidation Step>

FFCA acetal was obtained with a yield of 97% in the same manner as in Example 3, except that 5% palladium-supported carbon (Pd/C) (0.8 g, 10% by weight with respect to the substrate) was used as a catalyst. It is noted here that decomposition of neopentyl glycol was not confirmed in this step. The reaction conditions of the step (ii) are shown in Table 9.

<Step (iii): Deprotection Step>

FFCA was obtained with a yield of 95% in the same manner as in Example 1, except that the amount of water was changed to 6.0 g. It is noted here that decomposition of neopentyl glycol was not confirmed in this step. The reaction conditions of the step (iii) are shown in Table 10.

<Step (iv): Second Oxidation Step>

FDCA was obtained with a yield of 97% in the same manner as in Example 3, except that 5% palladium-supported carbon (Pd/C) (0.12 g, 10% by weight with respect to the substrate) was used as a catalyst. It is noted here that the overall yield of FDCA was 87%. The reaction conditions of the step (iv) and the yield of FDCA are shown in Table 11.

[Example C5]

<Step (i): Acetalization Step>

HMF acetal was obtained with a yield of 96% in the same manner as in Example 4, except that the amount of neopentyl glycol was changed to 39.6 g, 380.6 mmol. The reaction conditions of the step (i) are shown in Table 8.

<Step (ii): First Oxidation Step>

FFCA acetal was obtained with a yield of 97% in the same manner as in Example 3, except that 5% platinum-supported carbon (Pt/C) (0.8 g, 10% by weight with respect to the substrate) was used as a catalyst. It is noted here that decomposition of neopentyl glycol was not confirmed in this step. The reaction conditions of the step (ii) are shown in Table 9.

<Step (iii): Deprotection Step>

FFCA was obtained with a yield of 96% in the same manner as in Example 1, except that the amount of water was changed to 6.0 g and the reaction time was changed to 2 hours. It is noted here that decomposition of neopentyl glycol was not confirmed in this step. The reaction conditions of the step (iii) are shown in Table 10.

<Step (iv): Second Oxidation Step>

FDCA was obtained with a yield of 98% in the same manner as in Example 3, except that 5% platinum-supported carbon (Pt/C) (0.1 g, 10% by weight with respect to the substrate) was used as a catalyst. It is noted here that the overall yield of FDCA was 88%. The reaction conditions of the step (iv) and the yield of FDCA are shown in Table 11.

[Comparative Example C1]

HMF (0.3 g, 2.4 mmol) and 5% platinum-supported carbon (Pt/C) (0.03 g, 10% by weight with respect to the substrate) as a catalyst were added to water (2.7 g) used as a solvent. Air was introduced thereto at 0.9 MPa, and the resultant was stirred at 80°C for 16 hours. Subsequently, the resulting reaction solution was cooled to room temperature, and the pressure of the reaction vessel was reduced. Thereafter, the catalyst was separated by filtration, and sulfuric acid was poured into the thus obtained filtrate, after which the resulting precipitate was recovered by filtration. The thus obtained solid was washed with water to obtain FDCA with a yield of 22%. The reaction conditions and the yield of FDCA are shown in Table 12.

[Comparative Example C2]

FDCA was obtained with a yield of 57% in the same manner as in Comparative Example C1, except that HMF (0.9 g, 7.2 mmol), 5% platinum-supported carbon (Pt/C) (0.09 g, 10% by weight with respect to the substrate) as a catalyst, and potassium carbonate (2.0 g, 14.4 mmol) were added to water (2.1 g) used as a solvent. The reaction conditions and the yield of FDCA are shown in Table 12.

[Comparative Example C3]

FDCA was obtained with a yield of 45% in the same manner as in Comparative Example C1, except that HMF (1.2 g, 9.6 mmol), 5% platinum-supported carbon (Pt/C) (0.12 g, 10% by weight with respect to the substrate) as a catalyst, and potassium carbonate (2.65 g, 19.2 mmol) were added to water (1.8 g) used as a solvent. The reaction conditions and the yield of FDCA are shown in Table 12.

[Table 8]

| | HMF | | Substrate concentration (%) | Solvent(g) | Diol | | Catalyst | | Temperature (°C) | Time (h) |
|---|---|---|---|---|---|---|---|---|---|---|
| | Amount (g) | Number of moles(mmol) | | | Name | Amount (mmol) | Name | Amount with respect to substrate (wt%) | | |
| Example C1 | 10. 0 | 79.3 | 20 | Toluene 40.0 | Neopentyl glycol | 119.0 | AMBERLYST 15 | 10 | 30 | 1 |
| Example C2 | 10. 0 | 79.3 | 20 | Toluene 40.0 | Neopentyl glycol | 95.2 | AMBERLYST 15 | 10 | 30 | 1 |
| Example C3 | 40.0 | 317.2 | 40 | Propanediol 60.0 | Propanediol | 788.4 | - | - | 80 | 12 |
| Example C4 | 40.0 | 317.2 | 40 | Toluene 60.0 | Neopentyl glycol | 475.8 | AMBERLYST 15 | 10 | 30 | 1 |
| Example C5 | 40.0 | 317.2 | 40 | Toluene 60.0 | Neopentyl glycol | 380.6 | AMBERLYST 15 | 10 | 30 | 1 |

[Table 9]

| | HMF acetal | | Substrate concentration (%) | Solvent (g) | Base | | Catalyst | | Introduced air(MPa) | Temperature (°C) | Time (h) |
|---|---|---|---|---|---|---|---|---|---|---|---|
| | Amount (g) | Number of moles (mmol) | | | Name | Amount (mmol) | Name | Amount with respect to substrate (wt%) | | | |
| Example C1 | 4.0 | 18.8 | 20 | Water 16.0 | Potassium carbonate | 37.6 | Pd/C | 100 | 0.9 | 80 | 8 |
| Example C2 | 4.0 | 18.8 | 20 | Water 16.0 | Potassium carbonate | 37.6 | Pt/C | 100 | 0.9 | 80 | 8 |
| Example C3 | 8.0 | 37.7 | 40 | Water 12.0 | Potassium carbonate | 75.4 | Au/HAP | 10 | 0.9 | 80 | 8 |
| Example C4 | 8.0 | 37.7 | 40 | Water 12.0 | Potassium carbonate | 75.4 | Pd/C | 10 | 0.9 | 80 | 8 |
| Example C5 | 8.0 | 37.7 | 40 | Water 12.0 | Potassium carbonate | 75.4 | Pt/C | 10 | 0.9 | 80 | 8 |

[Table 10]

| | FFCA acetal | | Substrate concentration (%) | Solvent (g) | Catalyst | | Temperature (°C) | Time (h) |
|---|---|---|---|---|---|---|---|---|
| | Amount (g) | Number of moles (mmol) | | | Name | Amount with respect to substrate (wt%) | | |
| Example C1 | 4.0 | 17.7 | 20 | Water 16. 0 | RCP160M | 10 | 60 | 1 |
| Example C2 | 4.0 | 17.7 | 20 | Water 16. 0 | RCP160M | 10 | 60 | 1 |
| Example C3 | 4.0 | 17.7 | 40 | Water 6.0 | RCP160M | 10 | 60 | 2 |
| Example C4 | 4.0 | 17.7 | 40 | Water 6.0 | RCP160M | 10 | 60 | 1 |
| Example C5 | 4.0 | 17.7 | 40 | Water 6.0 | RCP160M | 10 | 60 | 2 |

[Table 11]

| | FFCA | | Substrate concentration (%) | Solvent (g) | Base | | Catalyst | | Introduced air (MPa) | Temperature (°C) | Time (h) | Overall yield of FDCA (%) |
|---|---|---|---|---|---|---|---|---|---|---|---|---|
| | Amount (g) | Number of moles (mmol) | | | Name | Amount (mmol) | Name | Amount with respect to substrate (wt%) | | | | |
| Example C1 | 2.4 | 17.0 | 20 | Water 9.6 | Potassium carbonate | 34.0 | Pd/C | 100 | 0.9 | 80 | 8 | 87 |
| Example C2 | 2.4 | 17.0 | 20 | Water 9.6 | Potassium carbonate | 34.0 | Pt/C | 100 | 0.9 | 80 | 8 | 89 |
| Example C3 | 1.2 | 8.6 | 40 | Water 1.8 | Potassium carbonate | 17.2 | Au/HAP | 10 | 0.9 | 80 | 8 | 80 |
| Example C4 | 1.2 | 8.6 | 40 | Water 1.8 | Potassium carbonate | 17.2 | Pd/C | 10 | 0.9 | 80 | 8 | 87 |
| Example C5 | 1.2 | 8.6 | 40 | Water 1.8 | Potassium carbonate | 17.2 | Pt/C | 10 | 0.9 | 80 | 8 | 88 |

[Table 12]

| | HMF | | Substrate concentration (%) | Solvent (g) | Base | | Catalyst | | Introduced air (MPa) | Temperature (°C) | Time (h) | Yield of FDCA (%) |
|---|---|---|---|---|---|---|---|---|---|---|---|---|
| | Amount (g) | Number of moles (mmol) | | | Name | Amount (mmol) | Name | Amount with respect to substrate (wt%) | | | | |
| Conparative Example C1 | 0.3 | 2. 4 | 10 | Water 2.7 | - | - | Pt/C | 10 | 0.9 | 80 | 16 | 22 |
| Conparative Example C2 | 0.9 | 7. 2 | 30 | Water 2.1 | Potassium carbonate | 14.4 | Pt/C | 10 | 0.9 | 80 | 16 | 57 |
| Conparative Example C3 | 1. 2 | 9. 6 | 40 | Water 1.8 | Potassium carbonate | 19.2 | Pt/C | 10 | 0.9 | 80 | 16 | 45 |

As shown in Table 11, in Examples C1 to C5 where a method including the steps (i) to (iv) was employed in the process of producing FDCA from HMF, FDCA was obtained with a high yield. On the other hand, as shown in Table 12, in Comparative Examples C1 to C3 where a conventional method of producing FDCA by direct oxidation of HMF was employed, the yield of FDCA was lower than in Examples C1 to C5.

## Claims

1. A method of producing 5-hydroxymethylfurfural, the method comprising the contact step of bringing glucose into contact with an isomerization catalyst and a dehydration catalyst in a biphasic solvent of water and a water-nonmiscible organic solvent.

2. A method of producing 5-hydroxymethylfurfural, the method comprising the contact step of bringing glucose into contact with an isomerization catalyst and a dehydration catalyst in a biphasic solvent of water and a water-nonmiscible organic solvent,
wherein the biphasic solvent comprises 80 parts by mass or less of an alkali metal salt or an alkaline earth metal salt with respect to 100 parts by mass of glucose.

3. The method of producing 5-hydroxymethylfurfural according to claim 1 or 2, wherein the isomerization catalyst is a metal oxide.

4. The method of producing 5-hydroxymethylfurfural according to any one of claims 1 to 3, wherein the dehydration catalyst is a solid acid.

5. The method of producing 5-hydroxymethylfurfural according to any one of claims 1 to 4, wherein

   the isomerization catalyst is aluminum oxide, and
   the dehydration catalyst is an ion exchange resin.

6. The method of producing 5-hydroxymethylfurfural according to any one of claims 1 to 5, wherein the organic solvent is hydrophobic and at least one selected from the group consisting of ethers, ketones, aromatic hydrocarbons, and saturated aliphatic hydrocarbons.

7. The method of producing 5-hydroxymethylfurfural according to any one of claims 1 to 6, wherein the organic solvent is at least one selected from the group consisting of methyltetrahydropyran (MTHP), methyl isobutyl ketone (MIBK), and methyl ethyl ketone (MEK).

8. The method of producing 5-hydroxymethylfurfural according to claim 1 or 2, further comprising the oil-water separation step of separating a reaction solution obtained by the contact step into an organic solvent phase comprising 5-hydroxymethylfurfural generated by the contact step, and an aqueous phase comprising a saccharide and the mixed catalyst that are contained in a solution obtained after the contact step.

9. The method of producing 5-hydroxymethylfurfural according to claim 8, comprising the recovery step of dissolving (distributing) and recovering 5-hydroxymethylfurfural generated by the contact step in the organic solvent phase obtained by the oil-water separation step.

10. The method of producing 5-hydroxymethylfurfural according to claim 8 or 9, comprising the step of recovering the saccharide and the mixed catalyst that are contained in the solution obtained after the contact step, along with the aqueous phase obtained by the oil-water separation step.

11. A method of producing a furan derivative comprising an acetal group and a carbonyl group, the method comprising the oxidation step of bringing hydroxymethylfurfural acetal into contact with an oxidation catalyst.

12. The method according to claim 11, wherein the furan derivative is a compound represented by Formula (1):

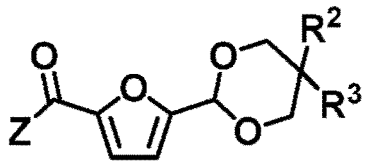 (1)

(wherein, Z represents a hydrogen atom or a hydroxyl group; and $R^2$ and $R^3$ are independently selected from a hydrogen atom, an alkyl group having 1 to 6 carbon atoms, and an aryl group having 6 to 12 carbon atoms that optionally has an alkyl substituent).

**13.** The method according to claim 11 or 12, wherein, in a product obtained after the oxidation step, the concentration of the furan derivative in all compounds having a furan skeleton is 70% by mole or higher.

**14.** The method according to any one of claims 11 to 13, wherein the oxidation catalyst is a metal catalyst.

**15.** A furan derivative represented by Formula (1):

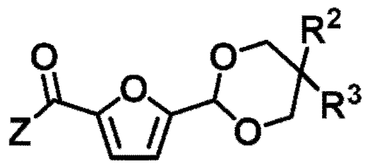 (1)

(wherein, Z represents a hydrogen atom or a hydroxyl group; and $R^2$ and $R^3$ are independently selected from a hydrogen atom, an alkyl group having 1 to 6 carbon atoms, and an aryl group having 6 to 12 carbon atoms that optionally has an alkyl substituent).

**16.** A solution comprising the furan derivative according to claim 15, wherein the concentration of the furan derivative in all compounds having a furan skeleton is 70% by mole or higher.

**17.** The solution according to claim 16, wherein the furan derivative is a compound represented by Formula (2):

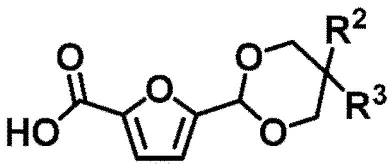 (2)

(wherein, $R^2$ and $R^3$ are independently selected from a hydrogen atom, an alkyl group having 1 to 6 carbon atoms, and an aryl group having 6 to 12 carbon atoms that optionally has an alkyl substituent).

**18.** A method of producing a furan derivative, the method comprising the deprotection step of deprotecting a formylfurancarboxylic acid acetal or an ester thereof to synthesize a furan derivative,
wherein the furan derivative is at least one selected from the group consisting of a formylfurancarboxylic acid and an ester thereof.

**19.** The method according to claim 18, wherein the formylfurancarboxylic acid acetal or ester thereof is a compound represented by Formula (3):

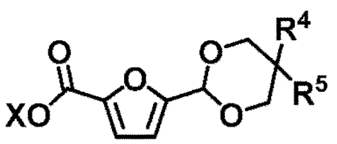 (3)

(wherein, X, $R^4$, and $R^5$ are independently selected from a hydrogen atom, an alkyl group having 1 to 6 carbon atoms, and an aryl group having 6 to 12 carbon atoms that optionally has an alkyl substituent).

**20.** The method according to claim 18 or 19, wherein, in a product obtained after the deprotection step, the concentration of the formylfurancarboxylic acid and ester thereof in all compounds having a furan skeleton is 70% by mole or higher.

**21.** The method according to any one of claims 18 to 20, comprising the recovery step of recovering a diol from a reaction solution after the deprotection step.

**22.** The method according to claim 21, wherein the diol is a diol having 2 to 10 carbon atoms.

**23.** A solution comprising compounds having a furan skeleton, wherein the concentration of a formylfurancarboxylic acid and an ester thereof in all of the compounds having a furan skeleton is 70% by mole or higher.

**24.** A method of producing a furandicarboxylic acid or an ester thereof, the method comprising the oxidation step of bringing a composition comprising a furan derivative into contact with an oxidation catalyst,
wherein

the furan derivative is at least one selected from the group consisting of diformylfuran, a formylfurancarboxylic acid, and an ester thereof, and
the concentration of the furan derivative in all compounds having a furan skeleton that are contained in the composition comprising the furan derivative is 70% by mole or higher.

**25.** The method according to claim 24, wherein the oxidation catalyst is a metal catalyst.

**26.** A method of producing a furandicarboxylic acid or a furandicarboxylic acid ester from hydroxymethylfurfural, the method comprising the following production steps (i) to (iv):

(i) the acetalization step of acetalizing hydroxymethylfurfural to synthesize hydroxymethylfurfural acetal;
(ii) the first oxidation step of oxidizing the hydroxymethylfurfural acetal to synthesize a formylfurancarboxylic acid acetal or an ester thereof;
(iii) the deprotection step of deprotecting the formylfurancarboxylic acid acetal or ester thereof to synthesize a formylfurancarboxylic acid or an ester thereof; and
(iv) the second oxidation step of oxidizing the formylfurancarboxylic acid or ester thereof.

**27.** The method of producing a furandicarboxylic acid or a furandicarboxylic acid ester according to claim 26, wherein the formylfurancarboxylic acid acetal or ester thereof is a compound represented by Formula (3):

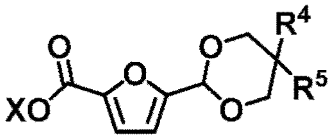 (3)

(wherein, X, $R^4$, and $R^5$ are independently selected from a hydrogen atom, an alkyl group having 1 to 6 carbon atoms, and an aryl group having 6 to 12 carbon atoms that optionally has an alkyl substituent).

**28.** The method of producing a furandicarboxylic acid or a furandicarboxylic acid ester according to claim 26 or 27, wherein, in the step (i), an organic solvent selected from toluene and methyltetrahydropyran is used.

**29.** The method of producing a furandicarboxylic acid or a furandicarboxylic acid ester according to any one of claims 26 to 28, comprising the recovery step of recovering a diol from a reaction solution after the step (iii).

**30.** The method of producing a furandicarboxylic acid or a furandicarboxylic acid ester according to claim 29, wherein the diol is a diol having 2 to 10 carbon atoms.

**INTERNATIONAL SEARCH REPORT**

International application No.
**PCT/JP2022/018549**

**A. CLASSIFICATION OF SUBJECT MATTER**

***C07D 307/46***(2006.01)i; ***C07D 307/68***(2006.01)i; ***C07D 407/04***(2006.01)i; *C07B 61/00*(2006.01)n
FI: C07D307/46; C07D407/04; C07D307/68; C07B61/00 300

According to International Patent Classification (IPC) or to both national classification and IPC

**B. FIELDS SEARCHED**

Minimum documentation searched (classification system followed by classification symbols)

C07D307/46; C07D307/68; C07D407/04; C07B61/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | PUMROD, S. et al., RSC Adv., 2020, vol. 10, pp. 9492-9498<br>abstract, p. 9494, left column, lines 1-13, p. 9495, left column, line 6 from the bottom to p. 9496, left column, line 2, p. 9497, right column, lines 23 -35 | 1-10 |
| Y | | 1-10 |
| A | | 11-30 |
| X | TORRES-OLEA,B. et al., Catalysts, 2020, vol. 10, no. 878, doi:10.3390/catal10080878<br>abstract, p. 9/21, lines 1-2, pp. 16/21 - 17/21 3.4. Catalytic Tests section, p. 17/21, 4. Conclusions section. | 1-4, 6-10 |
| Y | | 1-10 |
| A | | 11-30 |
| X | WO 2013/002397 A1 (NATIONAL UNIVERSITY CORPORATION EHIME UNIVERSITY) 03 January 2013 (2013-01-03)<br>examples 6-29 | 1, 2, 6-9 |
| Y | | 1-10 |
| A | | 11-30 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 July 2022** | **19 July 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/018549**

**C. DOCUMENTS CONSIDERED TO BE RELEVANT**

| Category* | Citation of document, with indication, where appropriate, of the relevant passages | Relevant to claim No. |
|---|---|---|
| Y | US 4339387 A (ROQUETTE FRERES) 13 July 1982 (1982-07-13) examples, claims | 1-10 |
| A | | 11-30 |
| X | WIESFELD,J.J. et al., Catalysis Symposium, Symposium A, preprints, 2020, vol. 126, ROMBUNNO. 3104 fig. 1, fig. 3, 2. Experimental Method section, 3. Result and Discussion section | 11-25 |
| Y | | 26-30 |
| A | | 1-10 |
| X | Catalysis Symposium, Symposium A preprints, 2019, vol. 124, ROMBUNNO. 1I10 fig. 1, 3, 2. Experiments section, 3. Results and Discussion section | 11-25 |
| Y | | 26-30 |
| A | | 1-10 |
| Y | KIM, M. et al., Angew. Chem. Int. Ed., 2018, vol. 57, pp. 8235-8239 p. 1, line 5 from the bottom to p. 2, line 24 | 26-30 |
| A | | 1-25 |
| Y | GREENE,T.W. et al., PROTECTIVE GROUPS IN ORGANIC SYNTHESIS THIRD EDITION, JOHN WILEY & SONS, INC., 1999, pp. 308, 309 p. 308, last line | 26-30 |
| A | | 1-25 |

Form PCT/ISA/210 (second sheet) (January 2015)

**INTERNATIONAL SEARCH REPORT**

International application No.
**PCT/JP2022/018549**

**Box No. III Observations where unity of invention is lacking (Continuation of item 3 of first sheet)**

This International Searching Authority found multiple inventions in this international application, as follows:

Claims 1-30 include a plurality of independent claims, and therefore, when examining the unity of invention, it is first necessary to consider the relationship between the independent claims rather than the dependent claims (Administrative Instructions Under the PCT Annex B(c)).

When the relationship among independent claims 1, 2, 11, 15, 18, 23, 24, and 26 is examined, it is found that the feature shared by these independent claims is a compound having a furan structure, such as 5-hydroxymethylfurfural, the compound represented by formulas (1), (2), (3), formylfuran dicarboxylic acid acetal, furan dicarboxylic acid.

However, these compounds having a furan structure are not disclosed only in patent document 5 (JP 2018-39778 A) described in paragraph [0009] of the present specification, but also in an extremely large number of documents such as documents (i) to (vi) below, and thus are not special technical features. Furthermore, there is no other identical or corresponding special technical feature between these independent claims.

(i) PUMROD, S. et al., RSC Adv., 2020, vol. 10, pp. 9492-9498 (abstract, p. 9494, left column, lines 1-14)
(ii) KIM, M. et al., Angew. Chem. Int. Ed., 2018, vol. 57, pp. 8235-8239 (abstract, table 1, fig. 2)
(iii) YANG C. et al., Fuel, 2020, vol. 278, https://doi.org/10.1016/j.fuel.2020.118361 (scheme 1, 2)
(iv) ZHOU, C. et al., ChemCatChem, 2015, vol. 7, pp. 2853-2863 (scheme 1, 2)
(v) CASANOVA. O. et al., Journal ofCatalysis, 2009, vol. 265, pp.109-116 (abstract)
(vi) KIM, M. et al., ACS Catal., 2019, vol. 9, pp. 4277-4285 (fig. 1, 3)

Therefore, claims 1-30 are classified into the following seven inventions.
(1) Claims 1-10
(2) Claim 11-14
(3) Claims 15-17
(4) Claims 18-22
(5) Claim 23
(6) Claims 24, 25
(7) Claims 26-30

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

**INTERNATIONAL SEARCH REPORT**
**Information on patent family members**

International application No.
**PCT/JP2022/018549**

| Patent document cited in search report | Publication date (day/month/year) | Patent family member(s) | Publication date (day/month/year) |
|---|---|---|---|
| WO 2013/002397 A1 | 03 January 2013 | (Family: none) | |
| US 4339387 A | 13 July 1982 | GB 2058070 A | |
| | | DE 3033527 A | |
| | | FR 2464260 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019089448 A
- JP 2009215172 A
- JP 5217142 B
- WO 2015155784 A
- JP 2018039778 A


### Non-patent literature cited in the description


- Chemical Engineering & Processing. Process Intensification. 2019, vol. 138, 65-72
- **YOSHIYUKI TAKASAKI.** Development of Isomerized Sugar by Glucose Isomerase. *The Society of Synthetic Organic Chemistry,* 1980, vol. 38 (6), 538-545
- *Royal Society of Chemistry Advances,* 2020, vol. 10, 9492-9498
- Green Chemistry. 2014, vol. 16, 4816-4838